# EUROPEAN PATENT SPECIFICATION

(11) **EP 1 460 128 B1**
(45) Date of publication and mention of the grant of the patent: **30.11.2016**
(21) Application number: 04004888.6
(22) Date of filing: 02.03.2004
(51) Int. Cl.: C12N 1/21, C12P 13/08, C12P 13/10, C12R 1/15

(54) **Method for producing L-arginine or L-lysine by fermentation**
Verfahren zur Herstellung von L-Arginin oder L-Lysin mittels Fermentation
Procédé de production de L-Arginine ou L-Lysine par fermentation

(30) Priority: 03.03.2003 JP 2003056129
(43) Date of publication of application: 22.09.2004
(73) Proprietor: Ajinomoto Co., Inc., Chuo-ku, Tokyo (JP)
(72) Inventor: Matsuzaki, Yumi, Kawasaki-shi Kanagawa (JP); Nakamura, Jun, Kawasaki-shi Kanagawa (JP); Hashiguchi, Kenichi, Kawasaki-shi Kanagawa (JP)
(74) Representative: Strehl Schübel-Hopf & Partner

(56) References cited:
- EP-A- 1 229 121
- SCHULZ A A ET AL: "NITROGEN AND CARBON REGULATION OF GLUTAMINE SYNTHETASE AND GLUTAMATE SYNTHASE IN CORYNEBACTERIUM GLUTAMICUM ATCC 13032" FEMS MICROBIOLOGY LETTERS, AMSTERDAM, NL, vol. 205, no. 2, 18 December 2001 (2001-12-18), pages 361-367, XP001070108 ISSN: 0378-1097
- JAKOBY M ET AL: "Isolation of the Corynebacterium glutamicum glnA gene encoding glutamine synthetase I" FEMS MICROBIOLOGY LETTERS, AMSTERDAM, NL, vol. 154, no. 1, 1997, pages 81-88, XP002226625 ISSN: 0378-1097
- JAKOBY M ET AL: "Nitrogen regulation in Corynebacterium glutamicum: Isolation of genes involved and biochemical characterization of corresponding proteins" FEMS MICROBIOLOGY LETTERS, AMSTERDAM, NL, vol. 173, no. 2, 15 April 1999 (1999-04-15), pages 303-310, XP002226626 ISSN: 0378-1097
- ARCONDÉGUY T ET AL: "PII signal transduction proteins, pivotal players in microbial nitrogen control" MICROBIOLOGY AND MOLECULAR BIOLOGY REVIEWS 2001 UNITED STATES, vol. 65, no. 1, 2001, pages 80-105, XP001182125 ISSN: 1092-2172

### SEQUENCE LISTING

<110> Ajinomoto. Co., Inc.
<120> Method for producing L-arginine or L-lysine by fermentation
<130> EPA-63398
<150> JP 2003-056129
   <151> 2003-03-03
<160> 24
<170> Patentln Ver. 2.0
<210> 1
   <211> 25
   <212> DNA
   <213> Artificial Sequence
<220>
   <223> Description of Artificial Sequence:primer for PCR
<400> 1
   cccgggtttt cttctgcaac tcggg 25
<210> 2
   <211> 25
   <212> DNA
   <213> Artificial Sequence
<220>
   <223> Description of Artificial Sequence:primer for PCR
<400> 2
   gtcgacaagc tcggttgttc ccagc 25
<210> 3
   <211> 24
   <212> DNA
   <213> Artificial Sequence
<220>
   <223> Description of Artificial Sequence:primer for PCR
<400> 3
   cccctagttc aaggcttgtt aatc 24
<210> 4
   <211> 25
   <212> DNA
   <213> Artificial Sequence
<220>
   <223> Description of Artificial Sequence:primer for PCR
<400> 4
   gtcttacctc ggctggttgg ccagc 25
<210> 5
   <211> 23
   <212> DNA
   <213> Artificial Sequence
<220>
   <223> Description of Artificial Sequence: primer
<400> 5
   agaactacga gtccgccttt ttg 23
<210> 6
   <211> 21
   <212> DNA
   <213> Artificial Sequence
<220>
   <223> Description of Artificial Sequence: primer
<400> 6
   cgatcaccag caacccacgc a 21
<210> 7
   <211> 22
   <212> DNA
   <213> Artificial Sequence
<220>
   <223> Description of Artificial Sequence: primer
<400> 7
   cttcccagta gcaccatacg ac 22
<210> 8
   <211> 26
   <212> DNA
   <213> Artificial Sequence
<220>
   <223> Description of Artificial Sequence: primer
<400> 8
   ctggtggcag ttcgaagagg tccttg 26
<210> 9
   <211> 26
   <212> DNA
   <213> Artificial Sequence
<220>
   <223> Description of Artificial Sequence: primer
<400> 9
   ggacaaggac ctcttcgaac tgccag 26
<210> 10
   <211> 26
   <212> DNA
   <213> Artificial Sequence
<220>
   <223> Description of Artificial Sequence: primer
<400> 10
   cggcgagacc gtcgattggg aggagc 26
<210> 11
   <211> 22
   <212> DNA
   <213> Artificial Sequence
<220>
   <223> Description of Artificial Sequence: primer
<400> 11
   gtagcacctt acgaccaaac cg 22
<210> 12
   <211> 20
   <212> DNA
   <213> Artificial Sequence
<220>
   <223> Description of Artificial Sequence: primer
<400> 12
   ggagccggtc gacgaggagc 20
<210> 13
   <211> 23
   <212> DNA
   <213> Artificial Sequence
<220>
   <223> Description of Artificial Sequence: primer
<400> 13
   gccccgggca ggcaagaatc ctc 23
<210> 14
   <211> 21
   <212> DNA
   <213> Artificial Sequence
<220>
   <223> Description of Artificial Sequence: primer
<400> 14
   tccccgggag gctctctgcg g 21
<210> 15
   <211> 4235
   <212> DNA
   <213> Brevibacteriun flavum
<220>
   <221> CDS
   <222> (1852).. (2364)
<400> 15
   aaacccgggt tttcttctgc aactcgggcg ccgaagcaaa cgaggctgct ttcaagattg 60
   cacgcttgac tggtcgttcc cggattctgg ctgcagttca tggtttccac ggccgcacca 120
   tgggttccct cgcgctgact ggccagccag acaagcgtga agcgttcctg ccaatgccaa 180
   gcggtgtgga gttctaccct tacggcgaca ccgattactt gcgcaaaatg gtagaaacca 240
   acccaacgga tgtggctgct atcttcctcg agccaatcca gggtgaaacg ggcgttgttc 300
   cagcacctga aggattcctc aaggcagtgc gcgagctgtg cgatgagtac ggcatcttga 360
   tgatcaccga tgaagtccag actggcgttg gccgtaccgg cgatttcttt gcacatcagc 420
   acgatggcgt tgttcccgat gtggtgacca tggccaaggg acttggcggc ggtcttccca 480
   tcggtgcttg tttggccact ggccgtgcag ctgaattgat gaccccaggc aagcacggca 540
   ccactttcgg tggcaaccca gttgcttgtg cagctgccaa ggcagtgctg tctgttgtcg 600
   atgacgcttt ctgcgcagaa gttacccgca agggcgagct gttcaaggta cttcttgcca 660
   aggttgacgg cgttgtagac gtccgtggca ggggcttgat gttgggcgtg gtgctggagc 720
   gcgacgtcgc aaagcaagct gttcttgatg gttttaagca cggcgttatt ttgaatgcac 780
   cggcggacaa cattatccgt ttgaccccgc cgctggtgat caccgacgaa gaaatcgcag 840
   acgcagtcaa ggctattgcc gagacaatcg cataaaggac ttaaacttat gacttcacaa 900
   ccacaggttc gccatttcct ggctgatgat gatctcaccc ctgcagagca ggcagaggtt 960
   ttgaccctag ccgcaaagct caaggcagcg ccgttttcgg agcgtccact cgagggacca 1020
   aagtccgttg cagttctttt tgataagact tcaactcgta ctcgcttctc cttcgacgcg 1080
   ggcatcgctc atttgggtgg acatgccatc gtcgtggatt ccggcagctc acagatgggt 1140
   aagggcgaga ccctgcagga caccgcagct gtattgtccc gctacgtgga agcaattgtg 1200
   tggcgcacct acgcacacag caatttccac gccatggcgg agacgtccac tgtgccgctg 1260
   gtgaactcct tgtccgatga tctgcaccca tgccagattc tggctgatct gcagaccatc 1320
   gtggaaaacc tcagccctga agaaggccca gcaggcctta agggtaagaa ggctgtgtac 1380
   ctgggcgatg gcgacaacaa catggccaac tcctacatga ttggctttgc caccgcgggc 1440
   atggatattt ccatcatcgc tcctgaaggg ttccagcctc gtgcggaatt cgtggagcgc 1500
   gcggaaaagc gtggccagga aaccggcgcg aaggttgttg tcaccgacag cctcgacgag 1560
   gttgccggcg ccgatgttgt catcaccgat acctgggtat ccatgggtat ggaaaacgac 1620
   ggcatcgatc gcaccacacc tttcgttcct taccaggtca acgatgaggt catggcgaaa 1680
   gctaacgacg gcgccatctt cctgcactgc cttcctgcct accgcggcaa agaagtggca 1740
   gcctccgtga ttgatggacc agcgtccaaa gttttcgatg aagcagaaaa ccgcctccac 1800
   gctcagaaag cactgctggt gtggctgctg gccaaccagc cgaggtaaga c atg tct 1857
   Met Ser
   1
   ctt ggc tca acc ccg tca aca ccg gaa aac tta aat ccc gtg act cgc 1905
   Leu Gly Ser Thr Pro Ser Thr Pro Glu Asn Leu Asn Pro Val Thr Arg
   5 10 15
   act gca cgc caa gct ctc att ttg cag att ttg gac aaa caa aaa gtc 1953
   Thr Ala Arg Gln Ala Leu Ile Leu Gln Ile Leu Asp Lys Gln Lys Val
   20 25 30
   acc agc cag gta caa ctg tct gaa ttg ctg ctg gat gaa ggc atc gat 2001
   Thr Ser Gln Val Gln Leu Ser Glu Leu Leu Leu Asp Glu Gly Ile Asp
   35 40 45 50
   atc acc cag gcc acc ttg tcc cgg gat ctc gat gaa ctc ggt gca cgc 2049
   Ile Thr Gln Ala Thr Leu Ser Arg Asp Leu Asp Glu Leu Gly Ala Arg
   55 60 65
   aag gtt cgc ccc gat ggg gga cgc gcc tac tac gcg gtc ggc cca gta 2097
   Lys Val Arg Pro Asp Gly Gly Arg Ala Tyr Tyr Ala Val Gly Pro Val
   70 75 80
   gat agc atc gcc cgc gaa gat ctc cgg ggt ccg tcg gag aag ctg cgc 2145
   Asp Ser Ile Ala Arg Glu Asp Leu Arg Gly Pro Ser Glu Lys Leu Arg
   85 90 95
   cgc atg ctt gat gaa ctg ctg gtt tct aca gat cat tcc ggc aac atc 2193
   Arg Met Leu Asp Glu Leu Leu Val Ser Thr Asp His Ser Gly Asn Ile
   100 105 110
   gcg atg ctg cgc acc ccg ccg gga gct gcc cag tac ctg gca agt ttc 2241
   Ala Met Leu Arg Thr Pro Pro Gly Ala Ala Gln Tyr Leu Ala Ser Phe
   115 120 125 130
   atc gat agg gtg ggg ctg aaa gaa gtc gtt ggc acc atc gct ggc gat 2289
   Ile Asp Arg Val Gly Leu Lys Glu Val Val Gly Thr Ile Ala Gly Asp
   135 140 145
   gac acc gtt ttt gtt ctc gcc cgt gat ccg ctc aca ggt aaa gaa cta 2337
   Asp Thr Val Phe Val Leu Ala Arg Asp Pro Leu Thr Gly Lys Glu Leu
   150 155 160
   ggt gaa tta ctc agc ggg cgc acc act taaagcgccc ctagttcaag 2384
   Gly Glu Leu Leu Ser Gly Arg Thr Thr
   165 170
   gcttgttaat cgcttgttaa tgcaggcagg taaggtataa cccgagtgtt ttttcgagga 2444
   ataccaaccc tttcaacaca ataattttct ttaaacatcc ttgctgtcca ccacggctgg 2504
   caaggaactt aaaatgaagg agcacacctc atgactaacc gcatcgttct tgcatactcc 2564
   ggcggtctgg acaccactgt ggcaattcca tacctgaaga agatgattga tggtgaagtc 2624
   atcgcagttt ctctcgacct gggccagggt ggagagaaca tggacaacgt tcgccagcgt 2684
   gcattggatg ccggtgcagc tgagtccatc gttgttgatg caaaggatga gttcgctgag 2744
   gagtactgcc tgccaaccat caaggcaaac ggcatgtaca tgaagcagta cccactggtt 2804
   tctgcaatct cccgcccact gatcgtcaag cacctcgttg aggctggcaa gcagttcaac 2864
   ggtacccacg ttgcacacgg ctgcactggt aagggcaacg accaggttcg tttcgaggtc 2924
   ggcttcatgg acaccgatcc aaacctggag atcattgcac ctgctcgtga cttcgcatgg 2984
   acccgcgaca aggctatcgc cttcgccgag gagaacaacg ttccaatcga gcagtccgtg 3044
   aagtccccat tctccatcga ccagaacgtc tggggccgcg ctattgagac cggttacctg 3104
   gaagatctgt ggaatgctcc aaccaaggac atctacgcat acaccgagga tccagctctg 3164
   ggtaacgctc cagatgaggt catcatctcc ttcgagggtg gcaagccagt ctccatcgat 3224
   ggccgtccag tctccgtact gcaggctatt gaagagctga accgtcgtgc aggcgcacag 3284
   ggcgttggcc gccttgacat ggttgaggac cgtctcgtgg gcatcaagtc ccgcgaaatc 3344
   tacgaagcac caggcgcaat cgcactgatt aaggctcacg aggctttgga agatgtcacc 3404
   atcgagcgcg aactggctcg ctacaagcgt ggcgttgacg cacgttgggc tgaggaagta 3464
   tacgacggcc tgtggttcgg acctctgaag cgctccctgg acgcgttcat tgattccacc 3524
   caggagcacg tcaccggcga tatccgcatg gttctgcacg caggttccat caccatcaat 3584
   ggtcgtcgtt ccagccactc cctgtacgac ttcaacctgg ctacctacga caccggcgac 3644
   accttcgacc agaccctggc taagggcttt gtccagctgc acggtctgtc ctccaagatc 3704
   gctaacaagc gcgatcgcga agctggcaac aactaagcca ccttttcaag catccagact 3764
   agaacttcaa gtatttagaa agtagaagaa caccacatgg aacagcacgg aaccaatgaa 3824
   ggtgcgctgt ggggcggccg cttctccggt ggaccctccg aggccatgtt cgccttgagt 3884
   gtctccactc atttcgactg ggttttggcc ccttatgatg tgttggcctc caaggcacac 3944
   gccaaggttt tgcaccaagc agagctactt tctgatgaag atctagccac catgctggct 4004
   ggtcttgatc agctgggcaa ggatgtcgcc gacggaacct tcggtccgct gccttctgat 4064
   gaggatgtgc acggcgcgat ggaacgcggt ctgattgacc gcgttggtcc tgaggtgggc 4124
   ggccgtctgc gcgctggtcg ttcccgcaac gaccaggtgg caaccctgtt ccgcatgtgg 4184
   gtccgcgacg cagtgcgcga catcgcgctg ggaacaaccg agcttgtcga c 4235
<210> 16
   <211> 171
   <212> PRT
   <213> Brevibacteriun flavum
<400> 16
   Met Ser Leu Gly Ser Thr Pro Ser Thr Pro Glu Asn Leu Asn Pro Val
   1 5 10 15
   Thr Arg Thr Ala Arg Gln Ala Leu Ile Leu Gln Ile Leu Asp Lys Gln
   20 25 30
   Lys Val Thr Ser Gln Val Gln Leu Ser Glu Leu Leu Leu Asp Glu Gly
   35 40 45
   Ile Asp Ile Thr Gln Ala Thr Leu Ser Arg Asp Leu Asp Glu Leu Gly
   50 55 60
   Ala Arg Lys Val Arg Pro Asp Gly Gly Arg Ala Tyr Tyr Ala Val Gly
   65 70 75 80
   Pro Val Asp Ser Ile Ala Arg Glu Asp Leu Arg Gly Pro Ser Glu Lys
   85 90 95
   Leu Arg Arg Met Leu Asp Glu Leu Leu Val Ser Thr Asp His Ser Gly
   100 105 110
   Asn Ile Ala Met Leu Arg Thr Pro Pro Gly Ala Ala Gln Tyr Leu Ala
   115 120 125
   Ser Phe Ile Asp Arg Val Gly Leu Lys Glu Val Val Gly Thr Ile Ala
   130 135 140
   Gly Asp Asp Thr Val Phe Val Leu Ala Arg Asp Pro Leu Thr Gly Lys
   145 150 155 160
   Glu Leu Gly Glu Leu Leu Ser Gly Arg Thr Thr
   165 170
<210> 17
   <211> 3138
   <212> DNA
   <213> Brevibacterium lactofermentum
<220>
   <221> CDS
   <222> (1).. (3138)
<400> 17
   atg tca gga ccg tta aga agt gaa cgt aaa gtc gtt ggc ttt gtc aga 48
   Met Ser Gly Pro Leu Arg Ser Glu Arg Lys Val Val Gly Phe Val Arg
   1 5 10 15
   gac cca ctg cca aaa gtt ggt tct tta tcg ctg aaa tct gag cat gcc 96
   Asp Pro Leu Pro Lys Val Gly Ser Leu Ser Leu Lys Ser Glu His Ala
   20 25 30
   caa gca gat cta gag cat ttg ggt tgg cgc aat gtt gag tct ttg gat 144
   Gln Ala Asp Leu Glu His Leu Gly Trp Arg Asn Val Glu Ser Leu Asp
   35 40 45
   ttg ttg tgg ggc ttg tca ggt gca ggc gat ccc gat gtc gcg ctg aac 192
   Leu Leu Trp Gly Leu Ser Gly Ala Gly Asp Pro Asp Val Ala Leu Asn
   50 55 60
   ctt ctt att cgg ctg tat cag gca ctt gaa gca atc ggc gag gat gct 240
   Leu Leu Ile Arg Leu Tyr Gln Ala Leu Glu Ala Ile Gly Glu Asp Ala
   65 70 75 80
   cga aac gag ctt gat caa gag att cgc cag gat gaa gaa cta cga gtc 288
   Arg Asn Glu Leu Asp Gln Glu Ile Arg Gln Asp Glu Glu Leu Arg Val
   85 90 95
   cgc ctt ttt gca ttg ttg ggt ggt tcc tcg gct gtc ggt gat cac ttg 336
   Arg Leu Phe Ala Leu Leu Gly Gly Ser Ser Ala Val Gly Asp His Leu
   100 105 110
   gtc gcc aat cct ttg cag tgg aaa ctc tta aaa ctt gat gcg cca tcg 384
   Val Ala Asn Pro Leu Gln Trp Lys Leu Leu Lys Leu Asp Ala Pro Ser
   115 120 125
   agg gaa gag atg ttt cag gcg ctg ctg gaa tct gtg aaa gct cag cct 432
   Arg Glu Glu Met Phe Gln Ala Leu Leu Glu Ser Val Lys Ala Gln Pro
   130 135 140
   gct gtg ctt gag gtt gag gat ttc agc gat gca cac aac att gcc cga 480
   Ala Val Leu Glu Val Glu Asp Phe Ser Asp Ala His Asn Ile Ala Arg
   145 150 155 160
   gac gat ttg agc acg cct ggt ttt tac acg gct agt gtt acc ggg cct 528
   Asp Asp Leu Ser Thr Pro Gly Phe Tyr Thr Ala Ser Val Thr Gly Pro
   165 170 175
   gaa gca gag cga gtc ttg aaa tgg act tat cgc acg ttg ctg acc cgg 576
   Glu Ala Glu Arg Val Leu Lys Trp Thr Tyr Arg Thr Leu Leu Thr Arg
   180 185 190
   att gct gcg cat gat tta gcg ggt acc tat ccc acc gac atg cgg aga 624
   Ile Ala Ala His Asp Leu Ala Gly Thr Tyr Pro Thr Asp Met Arg Arg
   195 200 205
   aaa ggt ggc gat cct gtt ccg ttt agc aca gtg acc atg cag ctc agc 672
   Lys Gly Gly Asp Pro Val Pro Phe Ser Thr Val Thr Met Gln Leu Ser
   210 215 220
   gac cta gct gat gct gct ttg act gct gct tta gct gtg gca att gcc 720
   Asp Leu Ala Asp Ala Ala Leu Thr Ala Ala Leu Ala Val Ala Ile Ala
   225 230 235 240
   aat gtt tat ggt gaa aag ccg gtt gat tca gct tta tct gtc atc gcg 768
   Asn Val Tyr Gly Glu Lys Pro Val Asp Ser Ala Leu Ser Val Ile Ala
   245 250 255
   atg ggc aaa tgt ggc gcg cag gaa ttg aac tac att tca gat gtg gac 816
   Met Gly Lys Cys Gly Ala Gln Glu Leu Asn Tyr Ile Ser Asp Val Asp
   260 265 270
   gtg gtg ttt gtt gca gag ccg gca aac tct aaa tca aca cgc acc gca 864
   Val Val Phe Val Ala Glu Pro Ala Asn Ser Lys Ser Thr Arg Thr Ala
   275 280 285
   gca gag ctc att cgc atc ggt agc aac tcg ttc ttt gag gtg gat gca 912
   Ala Glu Leu Ile Arg Ile Gly Ser Asn Ser Phe Phe Glu Val Asp Ala
   290 295 300
   gca ctt cgc cca gaa ggt aaa agt ggc gct ctt gtg cgc tct ttg gat 960
   Ala Leu Arg Pro Glu Gly Lys Ser Gly Ala Leu Val Arg Ser Leu Asp
   305 310 315 320
   tcc cat atg gcg tat tac aag cgc tgg gcg gaa acc tgg gaa ttt cag 1008
   Ser His Met Ala Tyr Tyr Lys Arg Trp Ala Glu Thr Trp Glu Phe Gln
   325 330 335
   gca ctg ctg aaa gct cgt ccc atg acg ggt gat att gac ctt ggg cag 1056
   Ala Leu Leu Lys Ala Arg Pro Met Thr Gly Asp Ile Asp Leu Gly Gln
   340 345 350
   tcc tat gtg gat gct ctt tca ccg ttg att tgg gcg gct agc cag cgg 1104
   Ser Tyr Val Asp Ala Leu Ser Pro Leu Ile Trp Ala Ala Ser Gln Arg
   355 360 365
   gaa tca ttt gtc aca gat gtc caa gct atg cgc cgt cga gtg ttg gac 1152
   Glu Ser Phe Val Thr Asp Val Gln Ala Met Arg Arg Arg Val Leu Asp
   370 375 380
   aat gtt ccg gaa gac ttg cgt gat cgt gag ctg aag ctt ggt cgc ggt 1200
   Asn Val Pro Glu Asp Leu Arg Asp Arg Glu Leu Lys Leu Gly Arg Gly
   385 390 395 400
   ggt ttg agg gat gtg gag ttt gct gtc cag ctc ctt cag atg gtg cat 1248
   Gly Leu Arg Asp Val Glu Phe Ala Val Gln Leu Leu Gln Met Val His
   405 410 415
   ggt cgc att gat gag acg ttg cgg gtt cgg tca acg gta aat gct ttg 1296
   Gly Arg Ile Asp Glu Thr Leu Arg Val Arg Ser Thr Val Asn Ala Leu
   420 425 430
   cat gtg ttg gtt gat cag gga tat gtg ggt cgt gaa gac ggg cat aat 1344
   His Val Leu Val Asp Gln Gly Tyr Val Gly Arg Glu Asp Gly His Asn
   435 440 445
   ctc att gag tcg tat gag ttt ttg cgc ctg ttg gag cat cgc ctt caa 1392
   Leu Ile Glu Ser Tyr Glu Phe Leu Arg Leu Leu Glu His Arg Leu Gln
   450 455 460
   ttg gag cgg atc aag cgc act cac ttg tta ccg aaa cct gat gac cga 1440
   Leu Glu Arg Ile Lys Arg Thr His Leu Leu Pro Lys Pro Asp Asp Arg
   465 470 475 480
   atg aat atg cgc tgg ttg gcg cgc gct tct ggg ttt act ggt tcg atg 1488
   Met Asn Met Arg Trp Leu Ala Arg Ala Ser Gly Phe Thr Gly Ser Met
   485 490 495
   gag caa agt tcg gcc aaa gct atg gaa cgg cat ttg cgt aag gtt cgt 1536
   Glu Gln Ser Ser Ala Lys Ala Met Glu Arg His Leu Arg Lys Val Arg
   500 505 510
   ttg cag att cag tcg ttg cat agt cag ctg ttt tat cgg cca ctg ctg 1584
   Leu Gln Ile Gln Ser Leu His Ser Gln Leu Phe Tyr Arg Pro Leu Leu
   515 520 525
   aac tct gtg gtc aac ttg agc gcg gat gcc atc aga ttg tct ccg gat 1632
   Asn Ser Val Val Asn Leu Ser Ala Asp Ala Ile Arg Leu Ser Pro Asp
   530 535 540
   gct gca aag cta caa ttg ggg gca ttg gga tac ctg cat cca tca cgt 1680
   Ala Ala Lys Leu Gln Leu Gly Ala Leu Gly Tyr Leu His Pro Ser Arg
   545 550 555 560
   gct tat gaa cac ctg act gct ctt gca tca gga gct agc cgt aaa gcc 1728
   Ala Tyr Glu His Leu Thr Ala Leu Ala Ser Gly Ala Ser Arg Lys Ala
   565 570 575
   aag att cag gcg atg ttg ctg ccc acg ttg atg gag tgg ctg tct caa 1776
   Lys Ile Gln Ala Met Leu Leu Pro Thr Leu Met Glu Trp Leu Ser Gln
   580 585 590
   aca gct gaa cca gat gcg gga ttg ctg aat tac cgc aag ctt tct gat 1824
   Thr Ala Glu Pro Asp Ala Gly Leu Leu Asn Tyr Arg Lys Leu Ser Asp
   595 600 605
   gct tcc tat gat cgc agc tgg ttt ttg cgc atg ctg cgt gat gag ggc 1872
   Ala Ser Tyr Asp Arg Ser Trp Phe Leu Arg Met Leu Arg Asp Glu Gly
   610 615 620
   gta gtg ggg cag cgg ttg atg cgt att ttg gga aat tct ccc tat att 1920
   Val Val Gly Gln Arg Leu Met Arg Ile Leu Gly Asn Ser Pro Tyr Ile
   625 630 635 640
   tct gaa ctg att atc tcc act ccg gac ttt gtg aaa cag ctg ggt gat 1968
   Ser Glu Leu Ile Ile Ser Thr Pro Asp Phe Val Lys Gln Leu Gly Asp
   645 650 655
   gcg gcg tct ggt cct aaa ttg ctt gct act gca ccg act cag gtt gtg 2016
   Ala Ala Ser Gly Pro Lys Leu Leu Ala Thr Ala Pro Thr Gln Val Val
   660 665 670
   aaa gca atc aag gcg acg gtg tcg cgt cat gag tca cct gat cgg gcg 2064
   Lys Ala Ile Lys Ala Thr Val Ser Arg His Glu Ser Pro Asp Arg Ala
   675 680 685
   atc cag gct gca cga tcg ctg agg agg cag gag ctg gca cgc att gcc 2112
   Ile Gln Ala Ala Arg Ser Leu Arg Arg Gln Glu Leu Ala Arg Ile Ala
   690 695 700
   tct gct gat ttg ctc aac atg ctc act gtt cag gaa gta tgc caa agc 2160
   Ser Ala Asp Leu Leu Asn Met Leu Thr Val Gln Glu Val Cys Gln Ser
   705 710 715 720
   ttg tca cta gtc tgg gat gcg gtg ttg gat gct gcc ttg gat gcg gaa 2208
   Leu Ser Leu Val Trp Asp Ala Val Leu Asp Ala Ala Leu Asp Ala Glu
   725 730 735
   atc cgt gct gca ctt aac gat cca cag aaa cca gat cag cct ctg gcc 2256
   Ile Arg Ala Ala Leu Asn Asp Pro Gln Lys Pro Asp Gln Pro Leu Ala
   740 745 750
   aat att tct gtg atc ggc atg ggc cgt ttg ggt gga gca gaa ctt gga 2304
   Asn Ile Ser Val Ile Gly Met Gly Arg Leu Gly Gly Ala Glu Leu Gly
   755 760 765
   tac ggt tct gat gcc gat gtg atg ttt gta tgc gag ccg gta gcc ggt 2352
   Tyr Gly Ser Asp Ala Asp Val Met Phe Val Cys Glu Pro Val Ala Gly
   770 775 780
   gtg gaa gag cat gag gcc gtc aca tgg tct att gcg atc tgt gat tcc 2400
   Val Glu Glu His Glu Ala Val Thr Trp Ser Ile Ala Ile Cys Asp Ser
   785 790 795 800
   atg cgg tcg agg ctt gcg cag cct tcc ggt gat cca cct ttg gag gtg 2448
   Met Arg Ser Arg Leu Ala Gln Pro Ser Gly Asp Pro Pro Leu Glu Val
   805 810 815
   gat ctg ggg ctg cgt cct gaa ggg aga tct ggt gcg att gtg cgc acc 2496
   Asp Leu Gly Leu Arg Pro Glu Gly Arg Ser Gly Ala Ile Val Arg Thr
   820 825 830
   gtt gat tcc tat gtg aag tac tac gaa aag tgg ggt gaa act tgg gag 2544
   Val Asp Ser Tyr Val Lys Tyr Tyr Glu Lys Trp Gly Glu Thr Trp Glu
   835 840 845
   att cag gcg ctg ctg agg gct gcg tgg gtt gct ggt gat cgt gag ctg 2592
   Ile Gln Ala Leu Leu Arg Ala Ala Trp Val Ala Gly Asp Arg Glu Leu
   850 855 860
   ggc att aag ttc ttg gag tcg att gat cgt ttc cgc tac cca gtt gac 2640
   Gly Ile Lys Phe Leu Glu Ser Ile Asp Arg Phe Arg Tyr Pro Val Asp
   865 870 875 880
   ggg gca acg cag gcg cag ctt cgt gaa gtt cgt cga att aag gcg agg 2688
   Gly Ala Thr Gln Ala Gln Leu Arg Glu Val Arg Arg Ile Lys Ala Arg
   885 890 895
   gtg gat aat gag agg ctt ccg cgc ggg gct gat cga aat acc cat acc 2736
   Val Asp Asn Glu Arg Leu Pro Arg Gly Ala Asp Arg Asn Thr His Thr
   900 905 910
   aag ctg ggt cgg gga gcg tta act gac atc gag tgg act gtg cag ttg 2784
   Lys Leu Gly Arg Gly Ala Leu Thr Asp Ile Glu Trp Thr Val Gln Leu
   915 920 925
   ttg acc atg atg cat gct cat gag att ccg gag ctg cac aat acg tcg 2832
   Leu Thr Met Met His Ala His Glu Ile Pro Glu Leu His Asn Thr Ser
   930 935 940
   acg ttg gaa gtt ctt gaa gtg ctg gaa aag cat cag att att aac cct 2880
   Thr Leu Glu Val Leu Glu Val Leu Glu Lys His Gln Ile Ile Asn Pro
   945 950 955 960
   gtg cag gtg cag acg ctt cgg gaa gcg tgg ctg acg gca acg gct gct 2928
   Val Gln Val Gln Thr Leu Arg Glu Ala Trp Leu Thr Ala Thr Ala Ala
   965 970 975
   agg aat gcg ctt gtg ctg gtc agg ggt aag aga tta gat cag tta cct 2976
   Arg Asn Ala Leu Val Leu Val Arg Gly Lys Arg Leu Asp Gln Leu Pro
   980 985 990
   act cct ggt ccg cac ctt gcg cag gtg gct ggt gcg tct ggt tgg gat 3024
   Thr Pro Gly Pro His Leu Ala Gln Val Ala Gly Ala Ser Gly Trp Asp
   995 1000 1005
   cca aat gag tac cag gag tat ttg gaa aac tat ctg aaa gtg acc agg 3072
   Pro Asn Glu Tyr Gln Glu Tyr Leu Glu Asn Tyr Leu Lys Val Thr Arg
   1010 1015 1020
   aag agt cgt cag gtt gtt gat gaa gtc ttc tgg ggt gtg gac tct atg 3120
   Lys Ser Arg Gln Val Val Asp Glu Val Phe Trp Gly Val Asp Ser Met
   1025 1030 1035 1040
   gag caa cgt gag ttt tag 3138
   Glu Gln Arg Glu Phe
   1045
<210> 18
   <211> 1045
   <212> PRT
   <213> Brevibacterium lactofermenturm
<400> 18
   Met Ser Gly Pro Leu Arg Ser Glu Arg Lys Val Val Gly Phe Val Arg
   1 5 10 15
   Asp Pro Leu Pro Lys Val Gly Ser Leu Ser Leu Lys Ser Glu His Ala
   20 25 30
   Gln Ala Asp Leu Glu His Leu Gly Trp Arg Asn Val Glu Ser Leu Asp
   35 40 45
   Leu Leu Trp Gly Leu Ser Gly Ala Gly Asp Pro Asp Val Ala Leu Asn
   50 55 60
   Leu Leu Ile Arg Leu Tyr Gln Ala Leu Glu Ala Ile Gly Glu Asp Ala
   65 70 75 80
   Arg Asn Glu Leu Asp Gln Glu Ile Arg Gln Asp Glu Glu Leu Arg Val
   85 90 95
   Arg Leu Phe Ala Leu Leu Gly Gly Ser Ser Ala Val Gly Asp His Leu
   100 105 110
   Val Ala Asn Pro Leu Gln Trp Lys Leu Leu Lys Leu Asp Ala Pro Ser
   115 120 125
   Arg Glu Glu Met Phe Gln Ala Leu Leu Glu Ser Val Lys Ala Gln Pro
   130 135 140
   Ala Val Leu Glu Val Glu Asp Phe Ser Asp Ala His Asn Ile Ala Arg
   145 150 155 160
   Asp Asp Leu Ser Thr Pro Gly Phe Tyr Thr Ala Ser Val Thr Gly Pro
   165 170 175
   Glu Ala Glu Arg Val Leu Lys Trp Thr Tyr Arg Thr Leu Leu Thr Arg
   180 185 190
   Ile Ala Ala His Asp Leu Ala Gly Thr Tyr Pro Thr Asp Met Arg Arg
   195 200 205
   Lys Gly Gly Asp Pro Val Pro Phe Ser Thr Val Thr Met Gln Leu Ser
   210 215 220
   Asp Leu Ala Asp Ala Ala Leu Thr Ala Ala Leu Ala Val Ala Ile Ala
   225 230 235 240
   Asn Val Tyr Gly Glu Lys Pro Val Asp Ser Ala Leu Ser Val Ile Ala
   245 250 255
   Met Gly Lys Cys Gly Ala Gln Glu Leu Asn Tyr Ile Ser Asp Val Asp
   260 265 270
   Val Val Phe Val Ala Glu Pro Ala Asn Ser Lys Ser Thr Arg Thr Ala
   275 280 285
   Ala Glu Leu Ile Arg Ile Gly Ser Asn Ser Phe Phe Glu Val Asp Ala
   290 295 300
   Ala Leu Arg Pro Glu Gly Lys Ser Gly Ala Leu Val Arg Ser Leu Asp
   305 310 315 320
   Ser His Met Ala Tyr Tyr Lys Arg Trp Ala Glu Thr Trp Glu Phe Gln
   325 330 335
   Ala Leu Leu Lys Ala Arg Pro Met Thr Gly Asp Ile Asp Leu Gly Gln
   340 345 350
   Ser Tyr Val Asp Ala Leu Ser Pro Leu Ile Trp Ala Ala Ser Gln Arg
   355 360 365
   Glu Ser Phe Val Thr Asp Val Gln Ala Met Arg Arg Arg Val Leu Asp
   370 375 380
   Asn Val Pro Glu Asp Leu Arg Asp Arg Glu Leu Lys Leu Gly Arg Gly
   385 390 395 400
   Gly Leu Arg Asp Val Glu Phe Ala Val Gln Leu Leu Gln Met Val His
   405 410 415
   Gly Arg Ile Asp Glu Thr Leu Arg Val Arg Ser Thr Val Asn Ala Leu
   420 425 430
   His Val Leu Val Asp Gln Gly Tyr Val Gly Arg Glu Asp Gly His Asn
   435 440 445
   Leu Ile Glu Ser Tyr Glu Phe Leu Arg Leu Leu Glu His Arg Leu Gln
   450 455 460
   Leu Glu Arg Ile Lys Arg Thr His Leu Leu Pro Lys Pro Asp Asp Arg
   465 470 475 480
   Met Asn Met Arg Trp Leu Ala Arg Ala Ser Gly Phe Thr Gly Ser Met
   485 490 495
   Glu Gln Ser Ser Ala Lys Ala Met Glu Arg His Leu Arg Lys Val Arg
   500 505 510
   Leu Gln Ile Gln Ser Leu His Ser Gln Leu Phe Tyr Arg Pro Leu Leu
   515 520 525
   Asn Ser Val Val Asn Leu Ser Ala Asp Ala Ile Arg Leu Ser Pro Asp
   530 535 540
   Ala Ala Lys Leu Gln Leu Gly Ala Leu Gly Tyr Leu His Pro Ser Arg
   545 550 555 560
   Ala Tyr Glu His Leu Thr Ala Leu Ala Ser Gly Ala Ser Arg Lys Ala
   565 570 575
   Lys Ile Gln Ala Met Leu Leu Pro Thr Leu Met Glu Trp Leu Ser Gln
   580 585 590
   Thr Ala Glu Pro Asp Ala Gly Leu Leu Asn Tyr Arg Lys Leu Ser Asp
   595 600 605
   Ala Ser Tyr Asp Arg Ser Trp Phe Leu Arg Met Leu Arg Asp Glu Gly
   610 615 620
   Val Val Gly Gln Arg Leu Met Arg Ile Leu Gly Asn Ser Pro Tyr Ile
   625 630 635 640
   Ser Glu Leu Ile Ile Ser Thr Pro Asp Phe Val Lys Gln Leu Gly Asp
   645 650 655
   Ala Ala Ser Gly Pro Lys Leu Leu Ala Thr Ala Pro Thr Gln Val Val
   660 665 670
   Lys Ala Ile Lys Ala Thr Val Ser Arg His Glu Ser Pro Asp Arg Ala
   675 680 685
   Ile Gln Ala Ala Arg Ser Leu Arg Arg Gln Glu Leu Ala Arg Ile Ala
   690 695 700
   Ser Ala Asp Leu Leu Asn Met Leu Thr Val Gln Glu Val Cys Gln Ser
   705 710 715 720
   Leu Ser Leu Val Trp Asp Ala Val Leu Asp Ala Ala Leu Asp Ala Glu
   725 730 735
   Ile Arg Ala Ala Leu Asn Asp Pro Gln Lys Pro Asp Gln Pro Leu Ala
   740 745 750
   Asn Ile Ser Val Ile Gly Met Gly Arg Leu Gly Gly Ala Glu Leu Gly
   755 760 765
   Tyr Gly Ser Asp Ala Asp Val Met Phe Val Cys Glu Pro Val Ala Gly
   770 775 780
   Val Glu Glu His Glu Ala Val Thr Trp Ser Ile Ala Ile Cys Asp Ser
   785 790 795 800
   Met Arg Ser Arg Leu Ala Gln Pro Ser Gly Asp Pro Pro Leu Glu Val
   805 810 815
   Asp Leu Gly Leu Arg Pro Glu Gly Arg Ser Gly Ala Ile Val Arg Thr
   820 825 830
   Val Asp Ser Tyr Val Lys Tyr Tyr Glu Lys Trp Gly Glu Thr Trp Glu
   835 840 845
   Ile Gln Ala Leu Leu Arg Ala Ala Trp Val Ala Gly Asp Arg Glu Leu
   850 855 860
   Gly Ile Lys Phe Leu Glu Ser Ile Asp Arg Phe Arg Tyr Pro Val Asp
   865 870 875 880
   Gly Ala Thr Gln Ala Gln Leu Arg Glu Val Arg Arg Ile Lys Ala Arg
   885 890 895
   Val Asp Asn Glu Arg Leu Pro Arg Gly Ala Asp Arg Asn Thr His Thr
   900 905 910
   Lys Leu Gly Arg Gly Ala Leu Thr Asp Ile Glu Trp Thr Val Gln Leu
   915 920 925
   Leu Thr Met Met His Ala His Glu Ile Pro Glu Leu His Asn Thr Ser
   930 935 940
   Thr Leu Glu Val Leu Glu Val Leu Glu Lys His Gln Ile Ile Asn Pro
   945 950 955 960
   Val Gln Val Gln Thr Leu Arg Glu Ala Trp Leu Thr Ala Thr Ala Ala
   965 970 975
   Arg Asn Ala Leu Val Leu Val Arg Gly Lys Arg Leu Asp Gln Leu Pro
   980 985 990
   Thr Pro Gly Pro His Leu Ala Gln Val Ala Gly Ala Ser Gly Trp Asp
   995 1000 1005
   Pro Asn Glu Tyr Gln Glu Tyr Leu Glu Asn Tyr Leu Lys Val Thr Arg
   1010 1015 1020
   Lys Ser Arg Gln Val Val Asp Glu Val Phe Trp Gly Val Asp Ser Met
   025 1030 1035 1040
   Glu Gln Arg Glu Phe
   1045
<210> 19
   <211> 1434
   <212> DNA
   <213> Brevibacteriun lactofermentum
<220>
   <221> CDS
   <222> (1)..(1434)
<400> 19
   gtg gcg ttt gaa acc ccg gaa gaa att gtc aag ttc atc aag gat gaa 48
   Val Ala Phe Glu Thr Pro Glu Glu Ile Val Lys Phe Ile Lys Asp Glu
   1 5 10 15
   aac gtc gag ttc gtt gac gtt cga ttc acc gac ctt ccc ggc acc gag 96
   Asn Val Glu Phe Val Asp Val Arg Phe Thr Asp Leu Pro Gly Thr Glu
   20 25 30
   cag cac ttc agc atc cca gct gcc agc ttc gat gca gat aca gtc gaa 144
   Gln His Phe Ser Ile Pro Ala Ala Ser Phe Asp Ala Asp Thr Val Glu
   35 40 45
   gaa ggt ctc gca ttc gac gga tcc tcg atc cgt ggc ttc acc acg atc 192
   Glu Gly Leu Ala Phe Asp Gly Ser Ser Ile Arg Gly Phe Thr Thr Ile
   50 55 60
   gac gaa tct gac atg aat ctc ctg cca gac ctc gga acg gcc acc ctt 240
   Asp Glu Ser Asp Met Asn Leu Leu Pro Asp Leu Gly Thr Ala Thr Leu
   65 70 75 80
   gat cca ttc cgc aag gca aag acc ctg aac gtt aag ttc ttc gtt cac 288
   Asp Pro Phe Arg Lys Ala Lys Thr Leu Asn Val Lys Phe Phe Val His
   85 90 95
   gat cct ttc acc cgc gag gca ttc tcc cgc gac cca cgc aac gta gca 336
   Asp Pro Phe Thr Arg Glu Ala Phe Ser Arg Asp Pro Arg Asn Val Ala
   100 105 110
   cgc aag gca gag cag tac ctg gca tcc acc ggc att gca gac acc tgc 384
   Arg Lys Ala Glu Gln Tyr Leu Ala Ser Thr Gly Ile Ala Asp Thr Cys
   115 120 125
   aac ttc ggc gcc gag gct gag ttc tac ctc ttc gac tcc gtt cgc tac    432
   Asn Phe Gly Ala Glu Ala Glu Phe Tyr Leu Phe Asp Ser Val Arg Tyr
       130                 135                 140
   tcc acc gag atg aac tcc ggc ttc tac gaa gta gat acc gaa gaa ggc    480
   Ser Thr Glu Met Asn Ser Gly Phe Tyr Glu Val Asp Thr Glu Glu Gly
   145                 150                 155                 160
   tgg tgg aac cgt ggc aag gaa acc aac ctc gac gga acc cca aac ctg    528
   Trp Trp Asn Arg Gly Lys Glu Thr Asn Leu Asp Gly Thr Pro Asn Leu
                   165                 170                 175
   ggc gca aag aac cgc gtc aag ggt ggc tac ttc cca gta gca cca tac    576
   Gly Ala Lys Asn Arg Val Lys Gly Gly Tyr Phe Pro Val Ala Pro Tyr
               180                 185                 190
   gac caa acc gtt gac gtg cgc gat gac atg gtt cgc aac ctc gca gct    624
   Asp Gln Thr Val Asp Val Arg Asp Asp Met Val Arg Asn Leu Ala Ala
           195                 200                 205
   tcc ggc ttc gct ctt gag cgt ttc cac cac gaa gtc ggt ggc gga cag    672
   Ser Gly Phe Ala Leu Glu Arg Phe His His Glu Val Gly Gly Gly Gln
       210                 215                 220
   cag gaa atc aac tac cgc ttc aac acc atg ctc cac gcg gca gat gat    720
   Gln Glu Ile Asn Tyr Arg Phe Asn Thr Met Leu His Ala Ala Asp Asp
   225                 230                 235                 240
   atc cag acc ttc aag tac atc atc aag aac acc gct cgc ctc cac ggc    768
   Ile Gln Thr Phe Lys Tyr Ile Ile Lys Asn Thr Ala Arg Leu His Gly
                   245                 250                 255
   aag gct gca acc ttc atg cct aag cca ctg gct ggc gac aac ggt tcc    816
   Lys Ala Ala Thr Phe Met Pro Lys Pro Leu Ala Gly Asp Asn Gly Ser
               260                 265                 270
   ggc atg cac gct cac cag tcc ctc tgg aag gac ggc aag cca ctc ttc    864
   Gly Met His Ala His Gln Ser Leu Trp Lys Asp Gly Lys Pro Leu Phe
           275                 280                 285
   cac gat gag tcc ggc tac gca ggc ctg tcc gac atc gcc cgc tac tac    912
   His Asp Glu Ser Gly Tyr Ala Gly Leu Ser Asp Ile Ala Arg Tyr Tyr
       290                 295                 300
   atc ggc ggc atc ctg cac cac gca ggc gct gtt ctg gcg ttc acc aac    960
   Ile Gly Gly Ile Leu His His Ala Gly Ala Val Leu Ala Phe Thr Asn
   305                 310                 315                 320
   gca acc ctg aac tcc tac cac cgt ctg gtt cca ggc ttc gag gct cca    1008
   Ala Thr Leu Asn Ser Tyr His Arg Leu Val Pro Gly Phe Glu Ala Pro
                   325                 330                 335
   atc aac ctg gtg tac tca cag cgc aac cgt tcc gct gct gtc cgt atc    1056
   Ile Asn Leu Val Tyr Ser Gln Arg Asn Arg Ser Ala Ala Val Arg Ile
               340                 345                 350
   cca atc acc gga tcc aac cca aag gca aag cgc atc gaa ttc cgc gct    1104
   Pro Ile Thr Gly Ser Asn Pro Lys Ala Lys Arg Ile Glu Phe Arg Ala
   355 360 365
   cca gac cca tca ggc aac cca tac ctg ggc ttc gca gcg atg atg atg 1152
   Pro Asp Pro Ser Gly Asn Pro Tyr Leu Gly Phe Ala Ala Met Met Met
   370 375 380
   gcc ggc ctc gac ggc atc aag aac cgc atc gag cca cac gct cca gtg 1200
   Ala Gly Leu Asp Gly Ile Lys Asn Arg Ile Glu Pro His Ala Pro Val
   385 390 395 400
   gac aag gac ctc tac gaa ctg cca cca gag gaa gct gca tcc att cca 1248
   Asp Lys Asp Leu Tyr Glu Leu Pro Pro Glu Glu Ala Ala Ser Ile Pro
   405 410 415
   cag gca cca acc tcc ctg gaa gca tcc ctg aag gca ctg cag gaa gac 1296
   Gln Ala Pro Thr Ser Leu Glu Ala Ser Leu Lys Ala Leu Gln Glu Asp
   420 425 430
   acc gac ttc ctc acc gag tct gac gtc ttc acc gag gat ctc atc gag 1344
   Thr Asp Phe Leu Thr Glu Ser Asp Val Phe Thr Glu Asp Leu Ile Glu
   435 440 445
   gcg tac atc cag tac aag tac gac aac gag atc tcc cca gtt cgc ctg 1392
   Ala Tyr Ile Gln Tyr Lys Tyr Asp Asn Glu Ile Ser Pro Val Arg Leu
   450 455 460
   cgc cca acc ccg cag gaa ttc gaa ttg tac ttc gac tgc taa 1434
   Arg Pro Thr Pro Gln Glu Phe Glu Leu Tyr Phe Asp Cys
   465 470 475
<210> 20
   <211> 477
   <212> PRT
   <213> Brevibacteriun lactofermentum
<400> 20
   Val Ala Phe Glu Thr Pro Glu Glu Ile Val Lys Phe Ile Lys Asp Glu
   1 5 10 15
   Asn Val Glu Phe Val Asp Val Arg Phe Thr Asp Leu Pro Gly Thr Glu
   20 25 30
   Gln His Phe Ser Ile Pro Ala Ala Ser Phe Asp Ala Asp Thr Val Glu
   35 40 45
   Glu Gly Leu Ala Phe Asp Gly Ser Ser Ile Arg Gly Phe Thr Thr Ile
   50 55 60
   Asp Glu Ser Asp Met Asn Leu Leu Pro Asp Leu Gly Thr Ala Thr Leu
   65 70 75 80
   Asp Pro Phe Arg Lys Ala Lys Thr Leu Asn Val Lys Phe Phe Val His
   85 90 95
   Asp Pro Phe Thr Arg Glu Ala Phe Ser Arg Asp Pro Arg Asn Val Ala
   100 105 110
   Arg Lys Ala Glu Gln Tyr Leu Ala Ser Thr Gly Ile Ala Asp Thr Cys
   115 120 125
   Asn Phe Gly Ala Glu Ala Glu Phe Tyr Leu Phe Asp Ser Val Arg Tyr
   130 135 140
   Ser Thr Glu Met Asn Ser Gly Phe Tyr Glu Val Asp Thr Glu Glu Gly
   145 150 155 160
   Trp Trp Asn Arg Gly Lys Glu Thr Asn Leu Asp Gly Thr Pro Asn Leu
   165 170 175
   Gly Ala Lys Asn Arg Val Lys Gly Gly Tyr Phe Pro Val Ala Pro Tyr
   180 185 190
   Asp Gln Thr Val Asp Val Arg Asp Asp Met Val Arg Asn Leu Ala Ala
   195 200 205
   Ser Gly Phe Ala Leu Glu Arg Phe His His Glu Val Gly Gly Gly Gln
   210 215 220
   Gln Glu Ile Asn Tyr Arg Phe Asn Thr Met Leu His Ala Ala Asp Asp
   225 230 235 240
   Ile Gln Thr Phe Lys Tyr Ile Ile Lys Asn Thr Ala Arg Leu His Gly
   245 250 255
   Lys Ala Ala Thr Phe Met Pro Lys Pro Leu Ala Gly Asp Asn Gly Ser
   260 265 270
   Gly Met His Ala His Gln Ser Leu Trp Lys Asp Gly Lys Pro Leu Phe
   275 280 285
   His Asp Glu Ser Gly Tyr Ala Gly Leu Ser Asp Ile Ala Arg Tyr Tyr
   290 295 300
   Ile Gly Gly Ile Leu His His Ala Gly Ala Val Leu Ala Phe Thr Asn
   305 310 315 320
   Ala Thr Leu Asn Ser Tyr His Arg Leu Val Pro Gly Phe Glu Ala Pro
   325 330 335
   Ile Asn Leu Val Tyr Ser Gln Arg Asn Arg Ser Ala Ala Val Arg Ile
   340 345 350
   Pro Ile Thr Gly Ser Asn Pro Lys Ala Lys Arg Ile Glu Phe Arg Ala
   355 360 365
   Pro Asp Pro Ser Gly Asn Pro Tyr Leu Gly Phe Ala Ala Met Met Met
   370 375 380
   Ala Gly Leu Asp Gly Ile Lys Asn Arg Ile Glu Pro His Ala Pro Val
   385 390 395 400
   Asp Lys Asp Leu Tyr Glu Leu Pro Pro Glu Glu Ala Ala Ser Ile Pro
   405 410 415
   Gln Ala Pro Thr Ser Leu Glu Ala Ser Leu Lys Ala Leu Gln Glu Asp
   420 425 430
   Thr Asp Phe Leu Thr Glu Ser Asp Val Phe Thr Glu Asp Leu Ile Glu
   435 440 445
   Ala Tyr Ile Gln Tyr Lys Tyr Asp Asn Glu Ile Ser Pro Val Arg Leu
   450 455 460
   Arg Pro Thr Pro Gln Glu Phe Glu Leu Tyr Phe Asp Cys
   465 470 475
<210> 21
   <211> 672
   <212> DNA
   <213> Brevibacteriun lactofermentum
<220>
   <221> CDS
   <222> (1).. (669)
<400> 21
   atg gca gga gca gtg gga cgc ccc cgg aga tca gct ccg cga cgg gca 48
   Met Ala Gly Ala Val Gly Arg Pro Arg Arg Ser Ala Pro Arg Arg Ala
   1 5 10 15
   ggc aag aat cct cgc gag gag att ctt gac gcc tct gct gag ctt ttc 96
   Gly Lys Asn Pro Arg Glu Glu Ile Leu Asp Ala Ser Ala Glu Leu Phe
   20 25 30
   acc cat caa ggc ttc gca aca acc tcc acg cat caa atc gct gat gcc 144
   Thr His Gln Gly Phe Ala Thr Thr Ser Thr His Gln Ile Ala Asp Ala
   35 40 45
   gtg gga atc cgc caa gcc tcg ctg tat tat cac ttc ccg tct aag acg 192
   Val Gly Ile Arg Gln Ala Ser Leu Tyr Tyr His Phe Pro Ser Lys Thr
   50 55 60
   gaa atc ttc ctc acc ctc ctg aaa tct acc gtc gag ccg tcc act gtg 240
   Glu Ile Phe Leu Thr Leu Leu Lys Ser Thr Val Glu Pro Ser Thr Val
   65 70 75 80
   ctc gcc gaa gac tta agc atc ctg gat gca gga cct gag atg cgc ctc 288
   Leu Ala Glu Asp Leu Ser Ile Leu Asp Ala Gly Pro Glu Met Arg Leu
   85 90 95
   tgg gca atc gtt gcc tcc gaa gtg cgt ctg ctg ctg tcc acc aag tgg 336
   Trp Ala Ile Val Ala Ser Glu Val Arg Leu Leu Leu Ser Thr Lys Trp
   100 105 110
   aac gtc ggt cgc ctg tac caa ctc ccc atc gtt ggt tct gaa gag ttc 384
   Asn Val Gly Arg Leu Tyr Gln Leu Pro Ile Val Gly Ser Glu Glu Phe
   115 120 125
   gcc gag tac cac agc cag cgc gaa gcc ctc acc aac atc ttc cgc gac 432
   Ala Glu Tyr His Ser Gln Arg Glu Ala Leu Thr Asn Ile Phe Arg Asp
   130 135 140
   ctc gcc acc gaa atc gtc ggt gac gac ccc cgc gca gaa ctc ccc ttc 480
   Leu Ala Thr Glu Ile Val Gly Asp Asp Pro Arg Ala Glu Leu Pro Phe
   145 150 155 160
   cac atc acc atg tcg gtg atc gaa atg cgt cgc aac gac ggc aag att 528
   His Ile Thr Met Ser Val Ile Glu Met Arg Arg Asn Asp Gly Lys Ile
   165 170 175
   cca agc ccg ctt tcc gca gac agc ctc ccg gag acc gca att atg ctt 576
   Pro Ser Pro Leu Ser Ala Asp Ser Leu Pro Glu Thr Ala Ile Met Leu
   180 185 190
   gcc gac gcc tcc ctc gcc gtc ctc ggc gcg tcg ctg ccc gcc gac cgg 624
   Ala Asp Ala Ser Leu Ala Val Leu Gly Ala Ser Leu Pro Ala Asp Arg
   195 200 205
   gtc gaa aaa acg ctt gaa cta atc aag cag gct gac gcg aaa taa cca 672
   Val Glu Lys Thr Leu Glu Leu Ile Lys Gln Ala Asp Ala Lys
   210 215 220
<210> 22
   <211> 222
   <212> PRT
   <213> Brev i bacter i un lactofermentum
<400> 22
   Met Ala Gly Ala Val Gly Arg Pro Arg Arg Ser Ala Pro Arg Arg Ala
   1 5 10 15
   Gly Lys Asn Pro Arg Glu Glu Ile Leu Asp Ala Ser Ala Glu Leu Phe
   20 25 30
   Thr His Gln Gly Phe Ala Thr Thr Ser Thr His Gln Ile Ala Asp Ala
   35 40 45
   Val Gly Ile Arg Gln Ala Ser Leu Tyr Tyr His Phe Pro Ser Lys Thr
   50 55 60
   Glu Ile Phe Leu Thr Leu Leu Lys Ser Thr Val Glu Pro Ser Thr Val
   65 70 75 80
   Leu Ala Glu Asp Leu Ser Ile Leu Asp Ala Gly Pro Glu Met Arg Leu
   85 90 95
   Trp Ala Ile Val Ala Ser Glu Val Arg Leu Leu Leu Ser Thr Lys Trp
   100 105 110
   Asn Val Gly Arg Leu Tyr Gln Leu Pro Ile Val Gly Ser Glu Glu Phe
   115 120 125
   Ala Glu Tyr His Ser Gln Arg Glu Ala Leu Thr Asn Ile Phe Arg Asp
   130 135 140
   Leu Ala Thr Glu Ile Val Gly Asp Asp Pro Arg Ala Glu Leu Pro Phe
   145 150 155 160
   His Ile Thr Met Ser Val Ile Glu Met Arg Arg Asn Asp Gly Lys Ile
   165 170 175
   Pro Ser Pro Leu Ser Ala Asp Ser Leu Pro Glu Thr Ala Ile Met Leu
   180 185 190
   Ala Asp Ala Ser Leu Ala Val Leu Gly Ala Ser Leu Pro Ala Asp Arg
   195 200 205
   Val Glu Lys Thr Leu Glu Leu Ile Lys Gln Ala Asp Ala Lys
   210 215 220
<210> 23
   <211> 2076
   <212> DNA
   <213> Brevibacteriun lactofermentum
<220>
   <221> CDS
   <222> (1).. (2076).
<400> 23
   atg aat aat cca gcc cag ctg cgc caa gat act gaa aag gaa gtc ctg 48
   Met Asn Asn Pro Ala Gln Leu Arg Gln Asp Thr Glu Lys Glu Val Leu
   1 5 10 15
   gcg ttg ctg ggc tct ttg gtt tta ccc gcc ggc acc gcg ctt gcc gcc 96
   Ala Leu Leu Gly Ser Leu Val Leu Pro Ala Gly Thr Ala Leu Ala Ala
   20 25 30
   acc gga tct ttg gcc agg tcc gaa ctc acg ccg tat tcc gat ttg gac 144
   Thr Gly Ser Leu Ala Arg Ser Glu Leu Thr Pro Tyr Ser Asp Leu Asp
   35 40 45
   ctc att ttg atc cat cca cca ggg gca acc ccg gat ggc gtg gag gat 192
   Leu Ile Leu Ile His Pro Pro Gly Ala Thr Pro Asp Gly Val Glu Asp
   50 55 60
   ttg tgg tac ccg att tgg gac gca aaa aag cgc ctc gac tac tcc gtg 240
   Leu Trp Tyr Pro Ile Trp Asp Ala Lys Lys Arg Leu Asp Tyr Ser Val
   65 70 75 80
   cgc acc cca gat gag tgc gtg gct atg att tct gcg gat tcc act gca 288
   Arg Thr Pro Asp Glu Cys Val Ala Met Ile Ser Ala Asp Ser Thr Ala
   85 90 95
   gcc ctt gcc atg ctt gac ctg cga ttt att gct ggc gat gag gat ctg 336
   Ala Leu Ala Met Leu Asp Leu Arg Phe Ile Ala Gly Asp Glu Asp Leu
   100 105 110
   tgt gcc aaa acg cgc cgg cgc atc gtg gag aag tgg cgc cag gaa ctc 384
   Cys Ala Lys Thr Arg Arg Arg Ile Val Glu Lys Trp Arg Gln Glu Leu
   115 120 125
   aac aaa aac ttc gac gcc gtt gtg gac acc gcg att gcc cgt tgg cgc 432
   Asn Lys Asn Phe Asp Ala Val Val Asp Thr Ala Ile Ala Arg Trp Arg
   130 135 140
   cgc tcc gga ccc gtc gtg gca atg acg cgg cca gat ctt aaa cac ggc 480
   Arg Ser Gly Pro Val Val Ala Met Thr Arg Pro Asp Leu Lys His Gly
   145 150 155 160
   agg gga ggg ctg cgc gat ttc gaa ctg atc aag gcc ctc gcg ctc ggc 528
   Arg Gly Gly Leu Arg Asp Phe Glu Leu Ile Lys Ala Leu Ala Leu Gly
   165 170 175
   cac cta tgc aac gtt cca cag cta gat acg caa cac cag ctg ctt ctc 576
   His Leu Cys Asn Val Pro Gln Leu Asp Thr Gln His Gln Leu Leu Leu
   180 185 190
   gac gcc cgc acc ttg ctg cac gtc cac gcg cga cgc tcc cgc gac gtc 624
   Asp Ala Arg Thr Leu Leu His Val His Ala Arg Arg Ser Arg Asp Val
   195 200 205
   ctt gat ccc gaa ttt gcg gtg gat gtg gcc atg gat ttg ggc ttt gtt 672
   Leu Asp Pro Glu Phe Ala Val Asp Val Ala Met Asp Leu Gly Phe Val
   210 215 220
   gac cgc tat cac tta ggc cgg gag atc gcc gat gca gcc cgc gcc att 720
   Asp Arg Tyr His Leu Gly Arg Glu Ile Ala Asp Ala Ala Arg Ala Ile
   225 230 235 240
   gat gac ggc ctg acc acc gcg ctg gcc acc gcc cgt ggc att ttg cca 768
   Asp Asp Gly Leu Thr Thr Ala Leu Ala Thr Ala Arg Gly Ile Leu Pro
   245 250 255
   cgt cgc acg ggt ttt gct ttt agg aat gct tct cga cgc cca ctt gat 816
   Arg Arg Thr Gly Phe Ala Phe Arg Asn Ala Ser Arg Arg Pro Leu Asp
   260 265 270
   ctt gat gtc gtc gac gcc aac ggc act atc gaa ttg tcc aaa aaa cca 864
   Leu Asp Val Val Asp Ala Asn Gly Thr Ile Glu Leu Ser Lys Lys Pro
   275 280 285
   gat ctt aat gat ccc gca ctt cca ctt cga gtg gcc gca gcc gca gcg 912
   Asp Leu Asn Asp Pro Ala Leu Pro Leu Arg Val Ala Ala Ala Ala Ala
   290 295 300
   acc acc gga ctt ccg gtg gca gaa tca acc tgg gct cga ctt aat gaa 960
   Thr Thr Gly Leu Pro Val Ala Glu Ser Thr Trp Ala Arg Leu Asn Glu
   305 310 315 320
   tgc ccg cca ctt cct gag cca tgg cct gcc aat gca gca ggg gac ttc 1008
   Cys Pro Pro Leu Pro Glu Pro Trp Pro Ala Asn Ala Ala Gly Asp Phe
   325 330 335
   ttt cgg att ctc tcc agt ccg aaa aac tca cgc cga gtg gtg aaa aat 1056
   Phe Arg Ile Leu Ser Ser Pro Lys Asn Ser Arg Arg Val Val Lys Asn
   340 345 350
   atg gat cgc cac gga ttg tgg tcg cgt ttt gtt cca gaa tgg gac cgc 1104
   Met Asp Arg His Gly Leu Trp Ser Arg Phe Val Pro Glu Trp Asp Arg
   355 360 365
   atc aaa ggg ctt atg ccc cgt gaa ccc agc cat att tcc acc atc gat 1152
   Ile Lys Gly Leu Met Pro Arg Glu Pro Ser His Ile Ser Thr Ile Asp
   370 375 380
   gaa cat agt ctg aac act gtt gca gga tgt gcg cta gaa act gtg acc 1200
   Glu His Ser Leu Asn Thr Val Ala Gly Cys Ala Leu Glu Thr Val Thr
   385 390 395 400
   gtc gcg cgc ccc gat ctt tta gtt ttg gga gcc ttg tac cac gac att 1248
   Val Ala Arg Pro Asp Leu Leu Val Leu Gly Ala Leu Tyr His Asp Ile
   405 410 415
   ggc aag ggc ttc ccg cgt cca cac gaa caa gta ggt gca gag atg gtg 1296
   Gly Lys Gly Phe Pro Arg Pro His Glu Gln Val Gly Ala Glu Met Val
   420 425 430
   gcg agg gcc gcg agc cgc atg ggg ttg aac ctt cgc gat cgt gcc agc 1344
   Ala Arg Ala Ala Ser Arg Met Gly Leu Asn Leu Arg Asp Arg Ala Ser
   435 440 445
   gtg caa acg ctg gtc gcc gag cac acc gcg gtg gcc aaa atc gcc gcg 1392
   Val Gln Thr Leu Val Ala Glu His Thr Ala Val Ala Lys Ile Ala Ala
   450 455 460
   cgc ctt gat ccc tcc tcg gag ggc gcc gtc gat aag ctg ctt gat gct 1440
   Arg Leu Asp Pro Ser Ser Glu Gly Ala Val Asp Lys Leu Leu Asp Ala
   465 470 475 480
   gtt agg tat gac ctg gtg aca ttg aat ctg ctt gag gtg cta aca gaa 1488
   Val Arg Tyr Asp Leu Val Thr Leu Asn Leu Leu Glu Val Leu Thr Glu
   485 490 495
   gct gat gcg aaa gcc acg ggg cct ggc gta tgg acg gcg cgt ttg gag 1536
   Ala Asp Ala Lys Ala Thr Gly Pro Gly Val Trp Thr Ala Arg Leu Glu
   500 505 510
   cat gcg ctg cgg att gtg tgc aag cgt gcg cgt gat cgc ctc acc gat 1584
   His Ala Leu Arg Ile Val Cys Lys Arg Ala Arg Asp Arg Leu Thr Asp
   515 520 525
   att cgc ccg gtt gcg ccg atg att gcg ccg cgt agc gaa att ggt ttg 1632
   Ile Arg Pro Val Ala Pro Met Ile Ala Pro Arg Ser Glu Ile Gly Leu
   530 535 540
   gtg gaa cgc gat ggc gtg ttc aca gtg caa tgg cac ggc gaa gac tta 1680
   Val Glu Arg Asp Gly Val Phe Thr Val Gln Trp His Gly Glu Asp Leu
   545 550 555 560
   cat cgg att ctt ggc gta att tat gcc aaa gga tgg aca atc acc gcg 1728
   His Arg Ile Leu Gly Val Ile Tyr Ala Lys Gly Trp Thr Ile Thr Ala
   565 570 575
   gcg cgc atg ctg gcc aat ggt caa tgg agt gcg gaa ttt gat gtc cgc 1776
   Ala Arg Met Leu Ala Asn Gly Gln Trp Ser Ala Glu Phe Asp Val Arg
   580 585 590
   gca aac ggc ccc caa gat ttt gat ccg cag cat ttc ctg cag gca tat 1824
   Ala Asn Gly Pro Gln Asp Phe Asp Pro Gln His Phe Leu Gln Ala Tyr
           595                 600                 605
   caa tcc ggt gtg ttt tcc gag gtt ccc att cca gca cct ggg ata aca    1872
   Gln Ser Gly Val Phe Ser Glu Val Pro Ile Pro Ala Pro Gly Ile Thr
       610                 615                 620
   gcc aca ttt tgg cac ggg aac act tta gaa gtg cgc act gag ctt cgc    1920
   Ala Thr Phe Trp His Gly Asn Thr Leu Glu Val Arg Thr Glu Leu Arg
   625                 630                 635                 640
   aca gga gct att ttt gcc ctg ctc aga aca ttg ccc gat gcc ctc tgg    1968
   Thr Gly Ala Ile Phe Ala Leu Leu Arg Thr Leu Pro Asp Ala Leu Trp
                   645                 650                 655
   atc aac gct gtg acc cgc ggt gcg acc ctg att atc cag gca gca ctg    2016
   Ile Asn Ala Val Thr Arg Gly Ala Thr Leu Ile Ile Gln Ala Ala Leu
               660                 665                 670
   aag ccc ggc ttc gat cga gca acg gtg gaa cgc tcc gta gtc agg tcg    2064
   Lys Pro Gly Phe Asp Arg Ala Thr Val Glu Arg Ser Val Val Arg Ser
           675                 680                 685
   ttg gca ggt agc                                                    2076
   Leu Ala Gly Ser
       690
<210> 24
   <211> 692
   <212> PRT
   <213> Brevibacterium lactofermentum
<400> 24
   Met Asn Asn Pro Ala Gln Leu Arg Gln Asp Thr Glu Lys Glu Val Leu
   1               5                   10                  15
   Ala Leu Leu Gly Ser Leu Val Leu Pro Ala Gly Thr Ala Leu Ala Ala
               20                  25                  30
   Thr Gly Ser Leu Ala Arg Ser Glu Leu Thr Pro Tyr Ser Asp Leu Asp
           35                  40                  45
   Leu Ile Leu Ile His Pro Pro Gly Ala Thr Pro Asp Gly Val Glu Asp
       50                  55                  60
   Leu Trp Tyr Pro Ile Trp Asp Ala Lys Lys Arg Leu Asp Tyr Ser Val
   65                  70                  75                  80
   Arg Thr Pro Asp Glu Cys Val Ala Met Ile Ser Ala Asp Ser Thr Ala
                   85                  90                  95
   Ala Leu Ala Met Leu Asp Leu Arg Phe Ile Ala Gly Asp Glu Asp Leu
               100                 105                 110
   Cys Ala Lys Thr Arg Arg Arg Ile Val Glu Lys Trp Arg Gln Glu Leu
           115                 120                 125
   Asn Lys Asn Phe Asp Ala Val Val Asp Thr Ala Ile Ala Arg Trp Arg
   130 135 140
   Arg Ser Gly Pro Val Val Ala Met Thr Arg Pro Asp Leu Lys His Gly
   145 150 155 160
   Arg Gly Gly Leu Arg Asp Phe Glu Leu Ile Lys Ala Leu Ala Leu Gly
   165 170 175
   His Leu Cys Asn Val Pro Gln Leu Asp Thr Gln His Gln Leu Leu Leu
   180 185 190
   Asp Ala Arg Thr Leu Leu His Val His Ala Arg Arg Ser Arg Asp Val
   195 200 205
   Leu Asp Pro Glu Phe Ala Val Asp Val Ala Met Asp Leu Gly Phe Val
   210 215 220
   Asp Arg Tyr His Leu Gly Arg Glu Ile Ala Asp Ala Ala Arg Ala Ile
   225 230 235 240
   Asp Asp Gly Leu Thr Thr Ala Leu Ala Thr Ala Arg Gly Ile Leu Pro
   245 250 255
   Arg Arg Thr Gly Phe Ala Phe Arg Asn Ala Ser Arg Arg Pro Leu Asp
   260 265 270
   Leu Asp Val Val Asp Ala Asn Gly Thr Ile Glu Leu Ser Lys Lys Pro
   275 280 285
   Asp Leu Asn Asp Pro Ala Leu Pro Leu Arg Val Ala Ala Ala Ala Ala
   290 295 300
   Thr Thr Gly Leu Pro Val Ala Glu Ser Thr Trp Ala Arg Leu Asn Glu
   305 310 315 320
   Cys Pro Pro Leu Pro Glu Pro Trp Pro Ala Asn Ala Ala Gly Asp Phe
   325 330 335
   Phe Arg Ile Leu Ser Ser Pro Lys Asn Ser Arg Arg Val Val Lys Asn
   340 345 350
   Met Asp Arg His Gly Leu Trp Ser Arg Phe Val Pro Glu Trp Asp Arg
   355 360 365
   Ile Lys Gly Leu Met Pro Arg Glu Pro Ser His Ile Ser Thr Ile Asp
   370 375 380
   Glu His Ser Leu Asn Thr Val Ala Gly Cys Ala Leu Glu Thr Val Thr
   385 390 395 400
   Val Ala Arg Pro Asp Leu Leu Val Leu Gly Ala Leu Tyr His Asp Ile
   405 410 415
   Gly Lys Gly Phe Pro Arg Pro His Glu Gln Val Gly Ala Glu Met Val
   420 425 430
   Ala Arg Ala Ala Ser Arg Met Gly Leu Asn Leu Arg Asp Arg Ala Ser
   435 440 445
   Val Gln Thr Leu Val Ala Glu His Thr Ala Val Ala Lys Ile Ala Ala
   450 455 460
   Arg Leu Asp Pro Ser Ser Glu Gly Ala Val Asp Lys Leu Leu Asp Ala
   465                 470                 475                 480
   Val Arg Tyr Asp Leu Val Thr Leu Asn Leu Leu Glu Val Leu Thr Glu
                   485                 490                 495
   Ala Asp Ala Lys Ala Thr Gly Pro Gly Val Trp Thr Ala Arg Leu Glu
               500                 505                 510
   His Ala Leu Arg Ile Val Cys Lys Arg Ala Arg Asp Arg Leu Thr Asp
           515                 520                 525
   Ile Arg Pro Val Ala Pro Met Ile Ala Pro Arg Ser Glu Ile Gly Leu
       530                 535                 540
   Val Glu Arg Asp Gly Val Phe Thr Val Gln Trp His Gly Glu Asp Leu
   545                 550                 555                 560
   His Arg Ile Leu Gly Val Ile Tyr Ala Lys Gly Trp Thr Ile Thr Ala
                   565                 570                 575
   Ala Arg Met Leu Ala Asn Gly Gln Trp Ser Ala Glu Phe Asp Val Arg
               580                 585                 590
   Ala Asn Gly Pro Gln Asp Phe Asp Pro Gln His Phe Leu Gln Ala Tyr
           595                 600                 605
   Gln Ser Gly Val Phe Ser Glu Val Pro Ile Pro Ala Pro Gly Ile Thr
       610                 615                 620
   Ala Thr Phe Trp His Gly Asn Thr Leu Glu Val Arg Thr Glu Leu Arg
   625                 630                 635                 640
   Thr Gly Ala Ile Phe Ala Leu Leu Arg Thr Leu Pro Asp Ala Leu Trp
                   645                 650                 655
   Ile Asn Ala Val Thr Arg Gly Ala Thr Leu Ile Ile Gln Ala Ala Leu
               660                 665                 670
   Lys Pro Gly Phe Asp Arg Ala Thr Val Glu Arg Ser Val Val Arg Ser
           675                 680                 685
   Leu Ala Gly Ser
       690

## Description

### BACKGROUND OF THE INVENTION

### Field of the Invention

The present invention relates to a method for producing L-arginine or
L-lysine, utilizing a coryneform bacterium. L-Arginine is an industrially useful amino acid as an ingredient of liver function-promoting agents, amino acid infusions, comprehensive amino acid pharmaceuticals and so forth, and L-lysine is also an industrially useful amino acid as an additive for animal feeds, component of health foods, amino acid infusions and so forth.

### Description of the Related Art

Breeding and improvement of microorganisms have been frequently used for production of an L-amino acid by fermentation. That is, since L-amino acid producing abilities of wild strains per se are extremely low in many cases, imparting auxotrophy or analogue resistance by mutation, imparting a metabolic regulation mutation, or utilizing a combination of these are known. According to such methods, L-amino acids can be obtained with appropriate yields. However, in order to industrially produce L-amino acids at a low cost, it is indispensable to further improve the fermentation yields.

It is considered that, in order to improve the fermentation yields, it is preferable to control an activity of enzyme in an amino acid biosynthetic pathway to be optimum, i.e., to enhance a biosynthesis system producing an amino acid from a carbon source.

Basic amino acids have a particularly high nitrogen content. Arginine molecule comprises 6 carbon atoms and 4 nitrogen atoms, and lysine molecule comprises 6 carbon atoms and 2 nitrogen atoms.

In amino acid fermentation, it is considered that the metabolism of nitrogen is important no less than the metabolism of carbon, and it is important to modify the metabolism of nitrogen as well as the metabolism of carbon in order to improve the fermentation yields. In the amino acid biosynthesis systems, a nitrogen atom is added by transamination of amino group contained in glutamic acid. Therefore, it is considered that increase of intracellular concentrations of glutamine and glutamic acid results in improvement of fermentation yields of amino acids.

Deletion of *odhA* gene encoding α-ketoglutarate dehydrogenase is conceived as a method for increasing glutamic acid concentration (WO95/34672), and enhancement of *glnA* gene encoding glutamine synthetase is conceived as a method for increasing glutamine concentration (European Patent Publication No. 1229121). Furthermore, it is disclosed that elimination of the adenylylation of glutamine synthetase is effective as a method for enhancing the L-glutamine supplying pathway (European Patent Publication No. 1229121).

However, for coryneform bacteria, influence of enhancement of the biosynthesis of L-glutamine or L-glutamic acid on the productivity of L-arginine or L-lysine is not known.

### SUMMARY OF THE INVENTION

It is an object of the present invention to improve fermentation yields of
L-arginine or L-lysine, by modifying the nitrogen metabolism of coryneform bacteria.

The present invention provides the following:
[1] A method for producing L-arginine or L-lysine, comprising the steps of
   (a) culturing a coryneform bacterium in a medium to produce and
   (b) accumulate L-arginine or L-lysine in the medium and
   (c) collecting the L-arginine or L-lysine from the medium,
   wherein said coryneform bacterium has an L-arginine- or L-lysine-producing ability and is modified so that glutamine synthetase activity is enhanced by
   (i) modifying an adenylylation site of glutamine synthetase so that activity regulation of glutamine synthetase by adenylylation is reduced compared with that of a wild strain or unmodified strain of a coryneform bacterium, or eliminated,
   (ii) disrupting a gene encoding the glutamine synthetase adenylyltransferase on a chromosome of said bacterium,
   (iii) disrupting a gene encoding PII protein on a chromosome, wherein the gene encoding PII protein is selected from the group consisting of:
      a) a DNA having the nucleotide sequence of SEQ ID NO: 23,
      b) a DNA encoding a protein having PII activity and being hybridizable with the nucleotide sequence of SEQ ID NO:23 under stringent conditions comprising washing at 0.1 x SSC, 0.1 % SDS, at 60°C,
   (iv) disrupting the *amtR* gene on a chromosome of said bacterium, wherein the amtR gene is selected from the group consisting of:
      a) a DNA having the nucleotide sequence of SEQ ID NO: 21,
      b) a DNA encoding a protein having AmtR activity and being hybridizable with the nucleotide sequence of SEQ ID NO:21 under stringent conditions comprising washing at 0.1 x SSC, 0.1 % SDS, at 60°C, or
   (v) enhancing expression of a gene encoding glutamine synthetase by replacing an expression regulatory sequence with a stronger one,
   and wherein said coryneform bacterium is modified so that the activity of the arginine repressor is reduced or eliminated as compared with a wild type strain or unmodified strain, wherein the arginine repressor is selected from the group consisting of:
   a) a protein encoded by a DNA having the nucleotide sequence of SEQ ID NO: 15,
   b) a protein having arginine repressor activity and being encoded by a DNA that is hybridizable with the nucleotide sequence of SEQ ID NO:15 under stringent conditions comprising washing at 0.1 x SSC, 0.1 % SDS, at 60°C.
[2] The method according to [1], wherein said gene encoding glutamine synthetase is selected from the group consisting of:
   a) a DNA that encodes the amino acid sequence of SEQ ID NO: 20,
   b) a DNA comprising the nucleotide sequence of SEQ ID NO: 19, and
   c) a DNA that is hybridizable with the nucleotide sequence of SEQ ID NO:19 under stringent conditions comprising washing at 0.1 x SSC, 0.1 % SDS, at 60°C, and encodes a protein having glutamine synthetase activity.

According to the present invention, in the production of L-arginine or L-lysine by fermentation using a coryneform bacterium, fermentation yield of L-arginine or L-lysine can be increased by modifying nitrogen metabolism regulation. Moreover, the DNA used in the present invention can be used for breeding of L-arginine or L-lysine producing coryneform bacteria.

### DESCRIPTION OF THE PREFERRED EMBODIMENTS

The inventors of the present invention conducted various researches in order to achieve the aforementioned object. As a result, they found that a strain of coryneform bacterium in which nitrogen metabolism regulating mechanism including the adenylylation of glutamine synthetase as a major part was modified showed superior fermentative production of
L-arginine and L-lysine, due to increase of intracellular concentrations of glutamine and glutamic acid.

Hereinafter, the present invention will be explained in detail.

Coryneform bacterium used for construction of the bacterium used in the present invention.

Coryneform bacteria include those bacteria having been hitherto classified into the genus *Brevibacterium* but united into the genus *Corynebacterium* at present (Int. J. Syst. Bacteriol., 41, 255 (1981)), and include bacteria belonging to the genus *Brevibacterium* closely relative to the genus *Corynebacterium.* Examples of such coryneform bacteria are listed below.
*Corynebacterium acetoacidophilum*
*Corynebacterium acetoglutamicum*
*Corynebacterium alkanolyticum*
*Corynebacterium callunae*
*Corynebacterium glutamicum*
*Corynebacterium lilium*
*Corynebacterium melassecola*
*Corynebacterium thermoaminogenes*
*Corynebacterium herculis*
*Brevibacterium divaricatum*
*Brevibacterium flavum*
*Brevibacterium immariophilum*
*Brevibacterium lactofermentum*
*Brevibacterium roseum*
*Brevibacterium saccharolyticum*
*Brevibacterium thiogenitalis*
*Corynebacterium ammoniagens*
*Brevibacterium album*
*Brevibacterium cerinum*
*Microbacterium ammoniaphilum*

Specifically, the following strains of these bacteria are exemplified:
*Corynebacterium acetoacidophilum* ATCC13870
*Corynebacterium acetoglutamicum* ATCC15806
*Corynebacterium alkanolyticum* ATCC21511
*Corynebacterium callunae* ATCC15991
*Corynebacterium glutamicum* ATCC13020, ATCC13032, ATCC13060
*Corynebacterium lilium* ATCC15990
*Corynebacterium melassecola* ATCC17965
*Corynebacterium efficiens* AJ12340 (FERM BP-1539)
*Corynebacterium herculis* ATCC13868
*Brevibacterium divaricatum* ATCC14020
*Brevibacterium flavum* ATCC13826, ATCC14067, AJ12418 (FERM BP-2205)
*Brevibacterium immariophilum* ATCC14068
*Brevibacterium lactofermentum* ATCC13869
*Brevibacterium roseum* ATCC13825
*Brevibacterium saccharolyticum* ATCC14066
*Brevibacterium thiogenitalis* ATCC19240
*Corynebacterium ammoniagenes* ATCC6871, ATCC6872
*Brevibacterium album* ATCC15111
*Brevibacterium cerinum* ATCC15112
*Microbacterium ammoniaphilum* ATCC15354

These strains can be obtained from, for example, the American Type Culture Collection (10801 University Boulevard, Manassas, VA 20110-2209, United States of America). That is, each strain is assigned its registration number, and one can request provision of each strain by utilizing its registration number. The registration numbers corresponding to the strains are indicated on the catalog of the American Type Culture Collection.

Furthermore, the AJ12340 strain was deposited on October 27, 1987 at the National Institute of Bioscience and Human-Technology, Agency of Industrial Science and Technology, Ministry of International Trade and Industry (currently, the independent administrative corporation, National Institute of Advanced Industrial Science and Technology, International Patent Organism Depositary (Chuo Dai-6, 1-1 Higashi 1-Chome, Tsukuba-shi, Ibaraki-ken, Japan, postal code: 305-5466)) as an international deposit under the provisions of the Budapest Treaty, and received an accession number of FERM BP-1539. The AJ12418 strain was deposited on January 5, 1989 at the National Institute of Bioscience and Human-Technology, Agency of Industrial Science and Technology, Ministry of International Trade and Industry as an internationaldeposit under the provisions of the Budapest Treaty and received an accession number of FERM BP-2205.

In the present invention, the term "L-arginine-producing ability" means an ability to cause accumulation of L-arginine in a medium when the bacterium is cultured in the medium. This L-arginine-producing ability may be a property of a wild strain of the coryneform bacterium, or a property imparted or enhanced by breeding.

In the present invention, the term "L-lysine-producing ability" means an ability to cause accumulation of L-lysine in a medium when the bacterium is cultured in the medium. This L-lysine-producing ability may be a property of a wild strain of the coryneform bacterium, or a property imparted or enhanced by breeding.

A corynefrom bacterium having L-lysine- or L-arginine-producing ability can be obtained by imparting an L-lysine- or L-arginine-producing ability to a wild-type strain of a corynefrom bacterium. Methods conventionally used for breeding of coryneform bacteria, *Escherichia* bacteria and so forth, can be used to impart the L-lysine- or L-arginine-producing ability. For example, such methods include, but are not limited to acquisition of auxotrophic mutant strains, analogue resistant strains or metabolic regulation mutant strains, creation of recombinant strains in which an L-lysine or L-arginine biosynthesis system enzyme is enhanced (see "Amino Acid Fermentation", the Japan Scientific Societies Press [Gakkai Shuppan Center], 1st Edition, published on May 30, 1986, pp.77 to 100) and so forth. Properties of auxotrophy, analogue resistance, metabolic regulation mutation and so forth may be individually imparted or two or more may be imparted in combination when breeding L-lysine- or L-arginine-producing bacteria. The biosynthesis system enzyme may be individually enhanced or two or more of them may be enhanced in combination. Furthermore, the impartation of properties including auxotrophy, analogue resistance, metabolic regulation mutation and so forth may be combined with the enhancement of biosynthesis system enzyme.

L-arginine-producing coryneform bacteria are not limited so long as they have an ability to produce L-arginine. Examples of such coryneform bacteria includes, but are not limited to, bacterium which is resistant to a certain agent, for example, a sulfa drug, 2-thiazolealanine, α-amino-β-hydroxyvaleric acid or the like; which is auxotrophic for L-histidine, L-proline, L-threonine, L-isoleucine, L-methionine or L-tryptophan in addition to resistant to 2-thiazolealanine (Japanese Patent Laid-open No. 54-44096); which is resistant to ketomalonic acid, fluoromalonic acid or monofluoroacetic acid (Japanese Patent Laid-open No. 57-18989), which is resistant to argininol (Japanese Patent Laid-open No. 62-24075); which is resistant to X-guanidine (X represents a derivative of fatty acid or aliphatic chain, Japanese Patent Laid-open No. 2-186995).

Coryneform bacterium producing L-arginine can be bred to be resistant to 5-azauracil, 6-azauracil, 2-thiouracil, 5-fluorouracil, 5-bromouracil, 5-azacytosine, 6-azacytosine and so forth; to be resistant to arginine hydroxamate and 2-thiouracil; to be resistant to arginine hydroxamate and 6-azauracil (see Japanese Patent Laid-open No. 49-126819); to be resistant to a histidine analogue or tryptophan analogue (see Japanese Patent Laid-open No. 52-114092); to be auxotrophic for at least one of methionine, histidine, threonine, proline, isoleucine, lysine, adenine, guanine and uracil (or uracil precursor) (see Japanese Patent Laid-open No. 52-99289); to be resistant to arginine hydroxamate (see Japanese Patent Publication No. 51-6754); to be auxotrophic for succinic acid or resistant to a nucleic acid base analogue (see Japanese Patent Laid-open No. 58-9692); to be unable to metabolize arginine and to be resistant to an arginine antagonist and canavanine and to be auxotorophic for lysine (see Japanese Patent Laid- open No. 52-8729); to be resistant to arginine, arginine hydroxamate, homoarginine, D-arginine and canavanine, or to be resistant to arginine hydroxamate and 6-azauracil (see Japanese Patent Laid-open No. 53-143288); to be resistant to canavanine (see Japanese Patent Laid-open No. 53-3586) and so forth.

Specific exmaples of L-arginine producing coryneform bacteria include the following strains.
*Brevibacterium flavum* AJ11169 (FERM P-4161)
*Brevibacterium lactofermentum* AJ12092 (FERM P-7273)
*Brevibacterium flavum* AJ11336 (FERM P-4939)
*Brevibacterium flavum* AJ11345 (FERM P-4948)
*Brevibacterium lactofermentum* AJ12430 (FERM BP-2228)

The AJ11169 strain and AJ12092 strain are 2-thiazolealanine resistant strains described in Japanese Patent Laid-open No. 54-44096, the AJ11336 strain is a strain having argininol resistance and sulfadiazine resistance described in Japanese Patent Publication (KOKOKU) No. No. 62-24075, the AJ11345 strain is a strain having argininol resistance, 2-thiazolealanine resistance, sulfaguanidine resistance and histidine auxotrophy described in Japanese Patent Publication No. No. 62-24075, and the AJ12430 strain is a strain having octylguanidine resistance and 2-thiazolealanine resistance described in Japanese Patent Laid-open No. 2-186995.

AJ11169 was deposited on August 3, 1977 at the National Institute of Bioscience and Human-Technology, Agency of Industrial Science and Technology, Ministry of International Trade and Industry (currently, the independent administrative corporation, National Institute of Advanced Industrial Science and Technology, International Patent Organism Depositary (Chuo Dai-6, 1-1 Higashi 1-Chome, Tsukuba-shi, Ibaraki-ken, Japan, postal code: 305-5466) and given an accession number of FERM P-4161. Then, it was converted to an international deposit based on the Budapest Treaty on September 27, 1999 and given an accession number of FERM BP-6892.

AJ12092 was deposited on September 29, 1983 at the National Institute of Bioscience and Human-Technology, Agency of Industrial Science and Technology, Ministry of International Trade and Industry and given an accession number of FERM P-7273. Then, it was converted to an international deposit based on the Budapest Treaty on October 1, 1999 and given an accession number of FERM BP-6906.

AJ11336 was deposited on April 25, 1979 at the National Institute of Bioscience and Human-Technology, Agency of Industrial Science and Technology, Ministry of International Trade and Industry and given an accession number of FERM P-4939. Then, it was converted to an international deposit based on the Budapest Treaty on September 27, 1999 and given an accession number of FERM BP-6893.

AJ11345 was deposited on April 25, 1979 at the National Institute of Bioscience and Human-Technology, Agency of Industrial Science and Technology, Ministry of International Trade and Industry and given an accession number of FERM P-4998. Then, it was converted to an international deposit based on the Budapest Treaty on September 27, 1999 and given an accession number of FERM BP-6894.

AJ12430 was deposited on December 26, 1988 the National Institute of Bioscience and Human-Technology, Agency of Industrial Science and Technology, Ministry of International Trade and Industry as an international deposit based on the Budapest Treaty, and given an accession number of FERM BP-2228.

Breeding to impart or enhance L-lysine productivity can be.performed by introducing mutation as follows. Such artificial mutants are as follows: S-(2-aminoethyl)-cysteine (hereinafter referred to as "AEC") resistant mutants: mutants requiring amino acids such as L-homoserine for their growth (see Japanese Patent Publication Nos. 4828078 and 566499); mutants resistant to AEC and requiring amino acids such as L-leucine, L-homoserine, L-proline, L-serine, L-arginine, L-alanine, L-valine, etc. (see U.S. Patents 3,708,395 and 3,825,472); L-lysine-producing mutants resistant to DL-α-amin-ε-caprolactam, α-aminolauryllactam, aspartic acid analogues, sulfa drugs, quinoids and N-lauroylleucine, and L-lysine-producing mutants resistant to oxaloacetate decarboxylase or respiratory system enzyme inhibitors (see Japanese Patent Laid-Open Nos. 5053588, 5031093, 52102498, 539394, 5386089, 559783, 559759, 5632995, 5639778, Japanese Patent Publication Nos. 5343591, 531833); L-lysine-producing mutants requiring inositol or acetic acid (see Japanese Patent Laid-Open Nos. 559784, 568692); L-lysine-producing mutants sensitive to fluoropyruvic acid or to temperatures of 34°C or higher (see Japanese Patent LaidOpen No. 5386090); L-lysine-producing mutants of bacteria belonging to the genus *Brevibacterum* or *Corynebacterium* resistant to ethylene glycol (see U.S. Patent 4,411,997).

Hereinafter, methods for imparting or enhancing the L-amino acid-producing ability by enhancing an L-arginine or L-lysine biosynthesis system enzyme activity are exemplified.

Enhancement of enzyme activity can be attained by introducing a mutation into a gene encoding the enzyme so that intracellular activity of the enzyme is enhanced, or by using a genetic recombination technique utilizing the gene.

The enzyme in L-arginine biosynthetic pathway may be one or more kinds of enzymes selected from N-acetylglutamyl phosphate reductase (*argC*), ornithine acetyltransferase (*argJ*), N-acetylglutamate kinase (*argB*), acetylornithine transaminase (*argD*), ornithine carbamoyltransferase (*argF*), argininosuccinate synthase (*argG*) and argininosucciniate lyase (*argH*), carbamoyl phosphate synthetase (*carAB*). The designations of the genes encoding these enzymes are indicated in the parentheses after the name of the enzymes, respectively.

Examples of the mutation that increases the activity of the protein encoded by the enzyme as described above include a mutation of a promoter sequence that increases transcription amount of the gene or a mutation in the coding region of the gene that increases specific activity of the enzyme protein and so forth.

Furthermore, as for enhancement of the enzymatic activity by using a genetic recombination technique, it can be attained by, for example, increasing copy number of a gene encoding the enzyme in a cell. For example, a DNA fragment containing the gene can be ligated to a vector functioning in a microorganism, preferably a multi-copy type vector, to prepare recombinant DNA and used to transform the microorganism.

The enhancement of gene expression can also be attained by, besides being based on the aforementioned gene amplification, replacing an expression regulatory sequence such as a promoter of the gene in a chromosomal DNA or plasmid with a stronger one (WO00/18935). Examples of strong promoters include lac promoter, trp promoter, trc promoter and so forth. Furthermore, by introducing nucleotide substitution or the like into the promoter region of the *lysE* gene, the promoter can be modified so as to become a stronger promoter. The substitution or modification of the promoter enhances expression of the gene. These modifications of the expression regulatory sequence can be combined with the increase of copy number of the gene.

Examples of the L-lysine biosynthesis system gene include, for example, a gene encoding the aspartokinase α-subunit protein or β-subunit protein for which synergistic feedback inhibition by L-lysine and L-threonine is desensitized (International Patent Publication WO94/25605), wild type phosphoenolpyruvate carboxylase gene derived from coryneform bacterium (Japanese Patent Laid-open No. 60-87788), gene encoding wild type dihydrodipicolinate synthetase derived from coryneform bacterium (Japanese Patent Publication No. 6-55149) and so forth.

The aforementioned methods for enhancing an activity of L-arginine biosynthesis system enzyme can be similarly applied to L-lysine.

Furthermore, by modifying a coryneform bacterium so that an arginine repressor is not function normally in the cell of the bacterium, an L-lysine- or L-arginine-producing ability can be imparted or enhanced.

Expression of an arginine biosynthesis system gene such as a gene of N-acetylglutamate synthase usually suffers marked supression by arginine in the medium. As for coryneform bacteria, it has been elucidated that production of some enzymes of the L-arginine biosynthesis system is supressed by L-arginine. Furthermore, it was reported that, while some of L-arginine biosynthesis system enzymes were suppressed by L-arginine, the suppression of these enzymes by L-arginine was eliminated in mutant strains of coryneform bacteria showing improved L-arginine accumulation amounts (Agric. Biol. Chem., 43(1), 105, 1979).

Furthermore, as for *Escherichia coli,* a repressor of L-arginine biosynthesis system (arginine repressor) and a gene encoding the repressor were identified (Proc. Natl. Acad. Sci. U.S.A. (1987), 84(19), 6697-701), and binding interactions of the repressor protein and various L-arginine biosynthesis system genes were also investigated (Proc. Natl. Acad. Sci. U.S.A. (1987), 84(19), 6697-701; J. Mol. Biol. (1992), 226, 367-386).

Arginine is produced through a biosynthetic pathway peculiar to arginine via intermediates such as ornithine and citrulline, and carbamylphosphate is taken up in this pathway. Therefore, it is considered necessary to enhance the carbamylphosphate synthesis pathway in order to increase the fermentation yield of arginine.

Carbamylphosphate is produced from a carbonate ion, glutamine and ATP. In coryneform bacteria, the carbonate ion is derived from culture broth, and ATP is generated in a metabolic process of saccharide. Therefore, supply of glutamine is important for production of carbamylphosphate.

Based on the above, it is considered that the L-lysine- and L-arginine-producing abilities can further be improved by combining the modification such that the arginine repressor is not function normally and increase of the glutamine synthetase activity described later.

In the present invention, the "arginine repressor" refers to a protein that has an effect of suppressing the L-arginine biosynthesis, and if expression amount of a gene that encodes this protein increases in a microorganism, L-arginine-producing ability will be reduced, and if the expression amount decreases or the protein disappears, the L-arginine-producing ability will be improved. Hereinafter, a gene encoding the arginine repressor is also called *argR* gene. The expression "arginine repressor does not function normally" means that the activity of the arginine repressor is reduced or eliminated as compared with a wild type strain or unmodified strain.

The nucleotide sequence of the *argR* gene of the *Brevibacterium flavum* and the amino acid encoded thereby are shown in SEQ ID NO: 15, and the amino acid sequence is shown in SEQ ID NO: 16.

In the present invention, the arginine repressor, which is an object of reducing the activity, may have an amino acid sequence including deletion, substitution, insertion or addition of one or more amino acid residues in the aforementioned amino acid sequence of the arginine repressor at one or more sites.

The number of "one or more" amino acid residues varies depending on the position of the amino acid residues in the three-dimensional structure of the protein and type of the amino acids. This is because of the following reason. That is, some of amino acids are highly homologous to one another, and difference within such amino acids does not significantly influence on the three-dimensional structure of, the protein. Thus, the mutant arginine repressor used in the present invention may be a protein exhibiting 30 to 50% or more, preferably 50 to 70% or more, more preferably 80 to 90% or more, most preferably 95% or more, of homology with respect to the whole amino acid residues constituting the arginine repressor and having the arginine repressor activity.

The DNA encoding a protein substantially identical to the aforementioned arginine repressor includes a DNA that is hybridizable with the *argR* gene under stringent conditions and encodes a protein having the arginine repressor activity. "Stringent condition" is a condition under which a so-called specific hybrid is formed, and a non-specific hybrid is not formed. It is difficult to clearly express this condition by using any numerical value.

In the present invention, the
stringent conditions include conditions whereby DNAs hybridize with each other at a salt concentration corresponding to a typical condition of Southern hybridization, i.e., 1 x SSC, 0.1% SDS, preferably 0.1 x SSC, 0.1% SDS, at 60°C.

Examples of the method for reducing the activity of arginine repressor of coryneform bacteria include, for example, treating a coryneform bacterium with ultraviolet ray irradiation or a mutagenesis agent used in a usual mutagenesis treatment such as N-methyl-N'-nitro-N-nitrosoguanidine (NTG) or nitrous acid and selecting a mutant strain showing reduced activity of arginine repressor. Besides the mutation treatment, a coryneform bacterium showing reduced activity of arginine repressor can also be obtained by transforming a coryneform bacterium with a DNA containing the *argR* gene encoding the arginine repressor modified so as not to produce arginine repressor that normally functions (deletion-type *argR* gene) by deleting a partial sequence of the *argR* gene to cause recombination between the deletion-type *argR* gene and the *argR* gene on a chromosome and thereby disrupt the *argR* gene on a chromosome.

The gene disruption concerning the *argR* gene can be carried out in the same manner as that for the gene disruption for the *glnE* gene described later.

The origin of the *argR* gene is not particularly limited, so long as it has homology in such a degree that it causes homologous recombination with the *argR* gene of a target microorganism. Specifically, examples of the *argR* genes of coryneform bacteria include, buta are not limited to, the *argR* gene of the *Brevibacterium flavum* described above and the *argR* gene of *Corynebacterium glutamicum* (GenBank accession AF049897). These *argR* genes are highly homologous, and therefore it is considered that even an *argR* gene of a coryneform bacterium of a genus or species different from that of the coryneform bacterium of which *argR* gene is to be disrupted may also be used for the gene disruption.

### Construction of coryneform bacterium used in the present invention

The coryneform bacterium used in the present invention is a coryneform bacterium which has an L-lysine- or L-arginine-producing ability as described above, and which is modified so that the intracellular glutamine synthetase (henceforth also referred to as "GS") activity is enhanced.

In breeding of the coryneform bacterium used in the present invention, either the impartation of the L-lysine- or L-arginine-producing ability or the enhancement of the GS activity may be carried out first.

The expression "modified so that the intracellular GS activity is enhanced" means that the GS activity per cell has become higher than that of an unmodified strain such as a wild-type coryneform bacterium. For example, those in which the number of GS molecules per cell is increased, those in which the GS activity per GS molecule is increased and so forth are encompassed. The "GS activity" means an activity of catalyzing a reaction of producing glutamine from glutamic acid and ammonia using ATP. Furthermore, the wild-type coryneform bacterium that serves as an object for comparison may be, for example, the *Brevibacterium lactofermentum* ATCC 13869.

Specifically, the coryneform bacterium which has enhanced GS activity is preferably, for example, a coryneform bacterium exhibiting a GS activity of 100 to 150 nmol/min/mg of intracellular protein or more, or a coryneform bacterium exhibiting a GS activity of 2 to 3 times higher than that of a wild strain. However, the coryneform bacterium used in the present invention is not limited to these. The GS activity can be measured by the method described in Journal of Fermentation and Bioengineering, Vol. 70, No. 3, 182-184, 1990. Furthermore, intracellular protein can be quantified by, for example, using Protein Assay (Bio-Rad) with bovine serum albumin standard.

Enhancement of the intracellular glutamine synthetase activity can be attained by, for example, enhancing expression of a gene encoding GS. Enhancement of the expression amount of the gene can be attained by increasing copy number of the gene encoding GS. For example, a gene fragment encoding GS is ligated to a vector functioning in the bacterium, preferably a multi-copy type vector, to prepare recombinant DNA and used to transform a coryneform bacterium.

Any of GS genes derived from coryneform bacteria or derived from other organisms such as *Escherichia* bacterium may be used. Among these, genes derived from coryneform bacteria are preferred in view of ease of expression.

The genes *glnA* (FEMS Microbiology Letters, 81-88 (154), 1997) and *glnA2* (Japanese Patent Laid-open No. 2002-300887, EP1229121, L. Nolden et al., FEMS Microbiology Letters, 201 (2001) 91-98) has already been reported as genes encoding GS of coryneform bacteria.

The nucleotide sequence of the *glnA* gene and the amino acid sequence encoded by the gene of *Brevibacterium lactofermentum* are shown in SEQ ID NO: 19, and the amino acid sequence is shown in SEQ ID NO: 20. Although the amino acid encoded by the initiation codon (nucleotide numbers 1 to 3) is described as valine in SEQ ID NOS: 19 and 20, it is highly possibly methionine. The nucleotide sequence of *glnA2* gene and the amino acid sequence encoded thereby are described in Japanese Patent Laid-open No. 2002-300887 and EP1229121.

In the present invention, GS may include deletion, substitution, insertion or addition of one or more amino acid residues at one or more sites so long as the GS activity is not reduced. The number of "two or more" amino acid residues varies depending on the position of the amino acid residues in the three-dimensional structure of the protein and type of amino acids. However, the mutant GS used in the present invention may be a protein exhibiting 30 to 50% or more, preferably 50 to 70% or more, more preferably 80 to 90% or more, most preferably 95% or more, of homology with respect to the whole amino acid residues constituting GS and having the GS activity.

The DNA encoding a protein substantially identical to the aforementioned GS includes a DNA that is hybridizable with the *glnA* or *glnA2* gene under stringent conditions and encodes a protein having the GS activity.

In the present invention, the stringent conditions include conditions whereby DNAs hybridize with each other at a salt concentration corresponding to an typical washing condition of Southern hybridization, i.e., 1 x SSC, 0.1% SDS, preferably 0.1 x SSC, 0.1% SDS, at 60°C.

Furthermore, the intracellular glutamine synthetase activity of coryneform bacterium can also be enhanced by modifying the bacterium so that glutamine synthetase adenylylation is reduced or eliminated.

GS changes into an inactive form by adenylylation of a tyrosine residue in its amino acid sequence (Proc. Natl. Acad. Sci. USA, 642-649, (58) 1967; J. Biol. Chem., 3769-3771, (243) 1968). Therefore, the intracellular GS activity can be enhanced by eliminating the activity control through the adenylylation of GS. The reduction or elimination of adenylylation referred to herein means not only substantially complete elimination of the adenylylation, but also such reduction of the adenylylation that the intracellular GS activity is enhanced. The "reduction" means that the adenylylation of GS is reduced compared with that of a wild strain or unmodified strain of a coryneform bacterium. The wild-type coryneform bacterium that serves as an object for comparison may be, for example, the *Brevibacterium lactofermentum* ATCC 13869.

Hereinafter, means for reducing or eliminating the activity control by adenylylation of GS are exemplified.

### Modification of adenylylation site of glutamine synthetase

In *Escherichia coli* etc., the adenylylation of GS is generally performed by glutamine synthetase adenylyltransferase (henceforth also referred to as "ATase', Proc. Natl. Acad. Sci. USA, 1703-1710, (58) 1967). Also in coryneform bacteria, GS is converted into its inactive form by adenylylation with glutamine synthetase adenylyltransferase (FEMS Microbiology Letters, 303-310, (173) 1999; FEMS Microbiology Letters, 201 (2001) 91-98). It has been suggested that, in coryneform bacteria, tyrosine residue at position 405 of the *glnA* gene product, which is represented by the sequence of Genebank accession Y13221, is adenylylated (FEMS Microbiology Letters, 303-310, (173) 1999). This inactivation by the adenylylation of GS can be eliminated by introducing a mutation into the *glnA* gene so that the tyrosine residue is replaced with another amino acid residue such as a phenylalanine residue.

Furthermore, it is expected that the aforementioned *glnA2* product also suffers from the activity control by adenylylation like the *glnA* product. Therefore, it is considered that the inactivation by the adenylylation of GS encoded by *glnA2* can be eliminated by introducing a mutation into the *glnA2* gene so that an amino acid residue corresponding to the tyrosine residue at position 405 is replaced with another amino acid residue such as a phenylalanine residue.

A DNA encoding GS of which activity control by adenylylation is eliminated can be obtained by modifying the sequence of *glnA* or *glnA2* so that the sequence includes such a mutation that the activity control by adenylylation of the encoded GS is eliminated. The obtained mutant gene can be introduced into a coryneform bacterium by any means similar to that used for the enhancement of L-arginine biosynthesis enzyme gene described above.

GS of which adenylylation site is modified may also have the amino acid sequence including deletion, substitution, insertion or addition of one or more amino acid residues at one or more sites in addition to the mutation eliminating the activity control by adenylylation so long as the GS activity is not reduced.

### Reduction of glutamine synthetase adenylyltransferase activity

The activity control of GS by the adenylylation can also be eliminated by reducing the activity of intracellular glutamine synthetase adenylyltransferase (ATase). The reduction of the ATase activity referred to herein means not only complete elimination of the activity but also such reduction of the activity that the intracellular ATase activity is reduced compared with that of a wild strain or unmodified strain of a coryneform bacterium. For example, those in which the copy number of ATase molecules per cell is reduced, those in which the ATase specific activity per ATase molecule is reduced and so forth are encompassed. Examples of the wild-type coryneform bacterium that serves as an object for comparison include, for example, the *Brevibacterium lactofermentum* ATCC 13869.

The gene *glnE* of the *Brevibacterium lactofermentum* ATCC 13869 strain has already been elucidated as a gene encoding ATase (EP1229121). The nucleotide sequence of the gene and the amino acid encoded thereby are shown in SEQ ID NO: 17, and the amino acid sequence is shown in SEQ ID NO: 18.

In the present invention, ATase, which is the object of the activity reduction, may have an amino acid sequence including deletion, substitution, insertion or addition of one or more amino acid residues in the aforementioned amino acid sequence of ATase at one or more sites.

The number of "two or more" amino acid residues varies depending on the position of the amino acid residues in the three-dimensional structure of the protein and type of amino acids. However, the mutant ATase used in the present invention may be a protein exhibiting 30 to 50% or more, preferably 50 to 70% or more, more preferably 80 to 90% or more, most preferably 95% or more, of homology with respect to the whole amino acid residues constituting ATase and having the ATase activity.

The DNA encoding a protein substantially identical to the aforementioned ATase includes a DNA that is hybridizable with the *glnE* gene under stringent conditions and codes for a protein having the ATase activity. "Stringent conditions" include a condition under which DNAs having high homology, for example, DNAs having homology of 50% or more, preferably 70% or more, more preferably 80% or more, most preferably 90% or more, hybridize with each other, but DNAs having homology lower than the above does not hybridize with each other. Alternatively, the stringent conditions include conditions whereby DNAs hybridize with each other at a salt concentration corresponding to an typical washing condition of Southern hybridization, i.e., 1 x SSC, 0.1% SDS, preferably 0.1 x SSC, 0.1% SDS, at 60°C.

Examples of the method for reducing the intracellular activity of ATase of coryneform bacteria include, for example, treating a coryneform bacterium with ultraviolet ray irradiation or a mutagenesis agent used in a usual mutagenesis treatment such as N-methyl-N'-nitro-N-nitrosoguanidine (NTG) or nitrous acid and selecting a mutant strain showing reduced activity of ATase. Besides the mutation treatment, a coryneform bacterium showing reduced activity of ATase can also be obtained by transforming a coryneform bacterium with a DNA containing the gene encoding ATase (*glnE*) modified so as not to produce ATase that normally functions (deletion-type *glnE* gene) by deleting a part of the *glnE* gene to cause recombination between the deletion-type *glnE* gene and the *glnE* gene on a chromosome and thereby disrupt the *glnE* gene on a chromosome. Such gene destruction by gene substitution utilizing homologous recombination has already been established, and there are methods of utilizing a linear DNA, a plasmid that contains a temperature sensitive replication origin and so forth.

A *glnE* gene on a host chromosome can be replaced with the deletion-type *glnE* gene, for example, as follows. That is, a recombinant DNA is prepared by inserting a temperature sensitive replication origin, a mutant *glnE* gene and a marker gene for resistance to a drug such as chloramphenicol, and used to transform a coryneform bacterium. Furthermore, the resultant transformant strain is cultured at a temperature at which the temperature sensitive replication origin does not function, and then the transformant strain can be cultured in a medium containing the drug to obtain a transformant strain in which the recombinant DNA is incorporated into a chromosomal DNA.

In such a strain in which the recombinant DNA is incorporated into a chromosomal DNA as described above, the mutant *glnE* gene is recombined with the *glnE* gene originally present on a chromosome, and the two fusion genes of the chromosomal *glnE* gene and the deletion-type *glnE* gene are inserted into the chromosome so that the other portions of the recombinant DNA (vector segment, temperature sensitive replication origin and drug resistance marker) is present between the two fusion genes.

In the *glnE* ligated to the plasmid, an internal sequence not containing any promoter and initiation codon is used. Therefore, the structural gene of GlnE is interrupted with the structure where the other portions of the recombinant DNA (vector segment, temperature sensitive replication origin and drug resistance marker) is present between the two fusion genes, and hence GlnE loses the function.

Furthermore, *glnE* of which internal sequence is deleted may also be used as the deletion-type *glnE.* In such a case, the native *glnE* gene is dominant in a state that two of fusion genes of the chromosomal *glnE* and the deletion-type *glnE* are inserted into the chromosomal DNA, and therefore the transformant strain expresses normal ATase. Then, in order to leave only the deletion-type *glnE* gene on the chromosomal DNA, one copy of the *glnE* gene is eliminated along with the vector segment (including the temperature sensitive replication origin and the drug resistance marker) from the chromosomal DNA by recombination of two of the *glnE* genes. In this case, the native *glnE* gene is left on the chromosomal DNA, and the deletion-type *glnE* gene is excised from the chromosomal DNA, or to the contrary, the deletion-type *glnE* gene is left on the chromosomal DNA, and the native *glnE* gene is excised from the chromosome DNA. In the both cases, the excised DNA is retained in the cell as a plasmid when the cell is cultured at a temperature at which the temperature sensitive replication origin can function. Subsequently, if the cell is cultured at a temperature at which the temperature sensitive replication origin cannot function, the *glnE* gene on the plasmid is eliminated along with the plasmid from the cell. Then, a strain in which *glnE* gene is disrupted can be obtained by selecting a strain in which the deletion-type *glnE* gene is left on the chromosome using PCR, Southern hybridization or the like.

Alternatively, gene disruption can be performed using a plasmid which cannot autonomously replicate in a coryneform bacterium in place of abode-mentioned temperature sensitive plasmid. Examples of plasmids which cannot autonomously replicate in a coryneform bacterium include pHSG299 (Takara Shuzo) and pHSG399 (Takara Shuzo) and the like.

A strain having disrupted *glnE* can be obtained as described above.

The origin of the *glnE* gene used for the gene disruption is not particularly limited so long as the *glnE* gene has homology to the *glnE* gene originally contained in a coryneform bacterium in such a degree that homologous recombination is occur between them. For example, ATases of *Mycobacterium tuberculosis* (GenBank ACCESION Z70692) and ATase of *Streptomyces coelicolor* (GenBank ACCESSION Y17736) have homologies of 51.9% and 33.4% to the ATase of coryneform bacteria, respectively (Japanese Patent Laid-open No. 2002-300887, EP1229121).

### Reduction of PII protein activity

The inactivation of GS by the adenylylation can also be defected by reducing the intracellular activity of PII protein. It is known that the PII protein is also involved in the adenylylation of GS by ATase. The PII protein is a signal transfer protein for controlling the GS activity, and it is known to be uridylylated by uridylyl transferase (UTase). The uridylylated PII protein promotes deadenylylation of GS by ATase, and the deuridylylated PII protein promotes the adenylylation of GS by ATase.

It has been reported that GS is highly adenylylated in a UTase deficient strain (J. Bacteriology, 569-577, (134) 1978). This phenotype of excessive adenylylation is suppressed by mutation of the PII protein (J. Bacteriology, 816-822, (164) 1985). That is, the inactivation of GS by the adenylylation can also be defected by reduction of PII protein activity. The reduction of PII protein activity means reduction of the function for promoting the adenylylation by ATase.

The reduction of the PII protein activity means, in addition to complete elimination of the activity, such reduction of the activity that the PII protein activity of the coryneform bacterium is lower than that of a wild strain or unmodified strain of the coryneform bacterium. For example, those in which the number of PII protein molecules per cell is reduced, those in which the specific activity of the PII protein per PII protein molecule is reduced and so forth are encompassed Examples of the wild-type coryneform bacterium that serves as an object for comparison include, for example, the *Brevibacterium lactofermentum* ATCC 13869. The *glnB* gene encoding the PII protein of coryneform bacteria has already been isolated, and it is suggested that the suppression of GS by the adenylylation of GS is defected by deletion of the gene (FEMS Microbiology Letters, 303-310, (173) 1999).

The gene *glnB* of *Brevibacterium lactofermentum* has already been elucidated as a gene encoding the PII protein, (EP1229121). The nucleotide sequence of the gene and the amino acid encoded thereby are shown in SEQ ID NO: 23, and the amino acid sequence is shown in SEQ ID NO: 24.

In the present invention, the PII protein, which is the object of the activity reduction, may have an amino acid sequence including deletion, substitution, insertion or addition of one or more amino acid residues in the aforementioned amino acid sequence of the PII protein at one or more sites.

The number of "one or more" amino acid residues varies depending on the position of the amino acid residues in the three-dimensional structure of the protein and type of amino acids. This is because of the following reason. That is, some of amino acids are highly homologous to one another, and difference within such amino acids does not significantly influence on the three-dimensional structure of the protein. Thus, the mutant the PII protein used in the present invention may be a protein exhibiting 30 to 50% or more, preferably 50 to 70% or more, more preferably 80 to 90% or more, most preferably 95% or more, of homology with respect to the whole amino acid residues constituting the PII protein and having the PII protein activity.

The DNA encoding a protein substantially identical to the aforementioned PII protein includes a DNA that is hybridizable with the *glnB* gene under a stringent condition and codes for a protein having the PII protein activity. The aforementioned "stringent condition" is a condition under which a so-called specific hybrid is formed, and a non-specific hybrid is not formed.

In the present invention, the stringent conditions include conditions whereby DNAs hybridize with each other at a salt concentration corresponding to an typical washing condition of Southern hybridization, i.e., 1 x SSC, 0.1% SDS, preferably 0.1 x SSC, 0.1% SDS, at 60°C.

Examples of the method for reducing the PII protein activity of coryneform bacteria include, for example, a method of treating a coryneform bacterium with ultraviolet ray irradiation or a mutagenesis agent used in a usual mutagenesis treatment such as N-methyl-N'-nitro-N-nitrosoguanidine (NTG) or nitrous acid and selecting a mutant strain showing reduced activity of the PII protein. Besides the mutation treatment, a coryneform bacterium showing reduced activity of the PII protein can also be obtained by transforming a coryneform bacterium with a DNA containing the *glnB* gene encoding the PII protein modified so as not to produce a PII protein that normally functions (deletion-type *glnB* gene) by deleting a part of the *glnB* gene to cause recombination between the deletion-type *glnB* gene and the *glnB* gene on a chromosome and thereby disrupt the *glnB* gene on a chromosome.

Such gene destruction for the *glnB* gene can be carried out in the same manner as that for the aforementioned gene disruption of the *glnE* gene.

### (4) Modification of nitrogen metabolism mechanism

The activity control by the adenylylation of GS may be eliminated also when a nitrogen metabolism regulation protein does not function normally.

The "nitrogen metabolism regulation protein" is a factor involved in the mechanism of changing GS into an inactive type by the adenylylation of the tyrosine residue in the amino acid sequence of GS described above (nitrogen metabolism regulation mechanism comprising the adenylylation of glutamine synthetase as a major part), and includes a positive factor and a negative factor. The positive factor is a factor that increases the intracellular GS activity, and the negative factor is a factor that reduces the intracellular GS activity. The nitrogen metabolism regulation protein regulates not only GS but also an ammonium ion incorporation gene (amt, *amtB*). With increase of extracellular ammonium ion concentration, the nitrogen metabolism regulation protein reduces the activity of the incorporation gene such as *amt* and *amtB,* and thereby the incorporation of ammonium ions is suppressed.

It is known that, in *Escherichia coli,* when the intracellular glutamine concentration decreases, NRI, which is the positive factor of the nitrogen metabolism regulation protein, binds to the promoter regulating expression of the *glnD* gene, which codes for a uridylyl transferase (Utase), to increase the expression amount of *glnD,* and increase of uridylylated PII protein promotes the deadenylylation by ATase to increase the GS activity (Mol. Microbaiology, (1998) (2) 29, 431-447).

It is known that, in *Bacillus subtilis,* when the intracellular glutamine concentration decreases, *TnrA* or *GlnR,* which are the negative factors of the nitrogen metabolism regulation protein, dissociates from the promoter that regulates the expression of the *glnD* gene encoding a uridylyl transferase (Utase), to increase the expression amount of *glnD,* and increase of uridylylated PII protein promotes the deadenylylation by ATase to increase the GS activity.

The nitrogen metabolism control mechanism utilizing the adenylylation of GS as a major mechanism is modified by the modification of the nitrogen metabolism regulation protein, and thereby the GS activity can be constantly increased. When the nitrogen metabolism regulator gene is a positive factor, the GS activity can be constantly increased by enhancing the activity of the factor, and when it is a negative factor, the GS activity can be constantly increased by reducing the activity of the factor.

In coryneform bacteria, the nitrogen metabolism control mechanism comprising the adenylylation of GS as a major part is controlled by the *amtR* gene product (AmtR), which is a negative nitrogen metabolism regulation protein (Mol. Microbiol., 37(4):969-77, Aug. 2000). Therefore, the GS activity can be increased by modifying the *amtR* gene so that AmtR is not function normally. The expression "AmtR does not function normally" means that the activity of AmtR is eliminated or reduced compared with a wild strain or unmodified strain of coryneform bacterium, and as a result, the GS activity is increased.

The nucleotide sequence of *amtR* and the amino acid encoded thereby of *Brevibacterium lactofermentum* are shown in SEQ ID NO: 21, and the amino acid sequence is shown in SEQ ID NO: 22.

In the present invention, AmtR, which is the object of the activity reduction, may have an amino acid sequence including deletion, substitution, insertion or addition of one or more amino acid residues in the aforementioned amino acid sequence of AmtR at one or more sites.

The number of "one or more" amino acid residues varies depending on the position in the three-dimensional structure of the protein and type of amino acid residue. However, the mutant AmtR used in the present invention may be a protein exhibiting 30 to 50% or more, preferably 50 to 70% or more, more preferably 80 to 90% or more, most preferably 95% or more, of homology with respect to the whole amino acid residues constituting AmtR and having the AmtR activity.

The DNA encoding a protein substantially identical to the aforementioned AmtR includes a DNA that is hybridizable with the *amtR* gene under a stringent condition and codes for a protein having the AmtR activity. The aforementioned "stringent condition" is a condition under which a so-called specific hybrid is formed, and a non-specific hybrid is not formed.

In the present invention, the stringent conditions include conditions whereby DNAs hybridize with each other at a salt concentration corresponding to an typical washing condition of Southern hybridization, i.e., 1 x SSC, 0.1% SDS, preferably 0.1 x SSC, 0.1% SDS, at 60°C.

Examples of the method for modifying a coryneform bacterium so that AmtR does not function normally include, for example, treating a coryneform bacterium with ultraviolet ray irradiation or a mutagenesis agent used in a usual mutagenesis treatment such as N-methyl-N'-nitro-N-nitrosoguanidine (NTG) or nitrous acid and selecting a mutant strain showing reduced activity of AmtR. Besides the mutation treatment, a coryneform bacterium showing reduced activity of AmtR can also be obtained by transforming a coryneform bacterium with a DNA containing the *amtR* gene encoding AmtR modified so as not to produce AmtR that normally functions (deletion-type *amtR* gene) by deleting a part of the *amtR* gene to cause recombination between the deletion-type *amtR* gene and the *amtR* gene on a chromosome and thereby disrupt the *amtR* gene on a chromosome.

Such gene destruction for the *amtR* gene can be carried out in the same manner as that for the aforementioned gene disruption of the *glnE* gene.

The reduction or elimination of the adenylylation of GS may be attained by a combination of two or more of means selected from the mutation of GS such that GS is not be adenylylated, reduction of ATase activity, reduction of PII protein activity and modification of the nitrogen metabolism regulation protein described above.

### Production of L-arginine or L-lysine using microorganism used in the present invention

L-arginine or L-lysine can be efficiently produced by culturing a coryneform bacterium obtained as described above in a medium to produce and accumulate L-arginine or L-lysine in the medium and collecting the L-arginine or L-lysine from the medium.

Culture can be performed in a conventional manner using a typical medium containing a carbon source, nitrogen source and mineral salts as well as organic trace nutrients such as amino acids and vitamins, as required. Either a synthetic medium or a natural medium may be used. Any kinds of carbon source and nitrogen source may be used so long as they can be utilized by a strain to be cultured.

As the carbon source, sugars such as glucose, glycerol, fructose, sucrose, maltose, mannose, galactose, starch hydrolysate and molasses, and organic acids such as acetic acid and citric acid can be used. In addition, alcohols such as ethanol can also be used each alone or in a combination with other carbon sources.

As the organic trace nutrients, amino acids, vitamins, fatty acids, nucleic acids, those containing those substances such as peptone, casamino acid, yeast extract and soybean protein decomposition product and so forth can be used. When an auxotrophic mutant that requires an amino acid or the like for its growth is used, it is preferable to supplement the required nutrient.

As the mineral salts, phosphates, magnesium salts, calcium salts, iron salts, manganese salts and so forth can be used.

The culture is preferably performed under aerobic conditions. The culture temperature is controlled to be between 20 to 45°C, and pH to be between 5 to 9. When pH falls during the culture, the medium is neutralized by addition of calcium carbonate or with an alkali such as ammonia gas or the like. A substantial amount of L-arginine or L-lysine is accumulated in the culture broth after 10 hours to 120 hours of culture in such a manner as described above.

After completion of the culture, L-arginine or L-lysine can be collected from the fermentation broth by a typical method. For example, after the cells were removed from the culture broth, L-arginine or L-lysine can be collected by concentrating the broth to crystallize L-glutamine.

### Examples

Hereinafter, the present invention will be explained more specifically with reference to the following examples.

### Reference Example 1: Construction of coryneform bacterium deficient in arginine repressor

### <1> Construction of plasmid for argR disruption

PCR was performed using chromosome DNA of a *Brevibacterium flavum* wild strain, 2247 strain (AJ14067), as a template and the oligonucleotides having the nucleotide sequences shown in SEQ ID NO: 1 (corresponds to the nucleotide numbers 4-28 in SEQ ID NO: 15) and SEQ ID NO: 2 (complementary to the sequence of the nucleotide numbers 4235-4211 in SEQ ID NO: 15) as primers. PCR was performed for 30 cycles each consisting of reactions at 98°C for 10 seconds, 58°C for 1 minute and 72°C for 3 minutes using Pyrobest DNA polymerase (Takara Shuzo). The amplified fragment was inserted into the *Sma*I site in the multi-cloning site of cloning vector pHSG399.

In order to delete the whole ORF considered to encode the arginine repressor from the inserted DNA fragment, PCR was performed using the oligonucleotides having the nucleotide sequences shown in SEQ ID NO: 3 (corresponds to the nucleotide numbers 2372-2395 in SEQ ID NO: 15) and SEQ ID NO: 4 (complementary to the sequence of the nucleotide numbers 1851-1827 in SEQ ID NO: 15) as primers and pHSG399 inserted with the amplified fragment as a template. The PCR product was self-ligated to obtain pssER.

Then, a fragment obtained by digesting pssER with restriction enzymes *Sma*I and *SalI* and the temperature sensitive plasmid pSFKT2 (see US6,303,383) and digested with *SmaI* and *SalI* were ligated to obtain a plasmid, pssERT for *argR* disruption whose autonomous replication ability in coryneform bacteria became temperature sensitive.

### <2> Acquisition of arginine repressor deficient strain of coryneform bacterium by homologous recombination

The plasmid pssERT obtained as described above was introduced into a *Brevibacterium lactofermentum* wild strain, 2256 (ATCC13869). The plasmid was introduced by the electric pulse method (Japanese Patent Laid-open No. 2-207791). Because this plasmid showed temperature sensitive autonomous replication ability in *Brevibacterium lactofermentum,* only strains in which this plasmid was incorporated into the chromosome by homologous recombination could be selected as kanamycin resistant strains at 34°C, which was a temperature that did not allow replication of the plasmid. Strains in which the plasmid for *argR* disruption was incorporated into a chromosome were selected as kanamycin resistant strains on a CM2G plate (containing 10 g/L of polypeptone, 10 g/L of yeast extract, 5 g/L of glucose, 5 g/L of NaCl and 15 g/L of agar in 1 L of water, pH 7.2) containing 25 µg/ml of kanamycin. At this stage, native *argR* gene derived from the chromosome and the argG gene derived from the plasmid in which ORF was deleted were present in tandem at the both sides of the plasmid portion on the chromosome.

Then, the recombinant strains were allowed to cause homologous recombination again, and strains that became kanamycin sensitive were selected at 34°C, which was a temperature that did not allow the plasmid replication to obtain strains in which one of the *argR* genes was eliminated. These strains included strains in which the native *argR* gene remained on the chromosome and strains in which the disrupted *argR* gene remained on the chromosome. From these strains, a strain having only the disrupted *argR* gene was selected. An *argR* gene on the chromosome could be determined to be the disrupted-type by preparing chromosome of a strain that became kanamycin sensitive at 34°C, performing PCR utilizing the chromosome as a template and the oligonucleotides having the nucleotide sequences shown in SEQ ID NOS: 1 and 2 as primers, and confirming that the PCR product was shorter by about 600 bp than that obtained by similarly performing PCR utilizing chromosome derived from the parent strain as a template.

Direct sequencing of the PCR product of the *argR-*disrupted strain selected as described above was performed to confirm that the *argR* gene was disrupted as desired, and thus 2256DR stain was obtained. This strain produces a markedly larger amount of L-arginine compared with the parent strain (see US2002045223_A1).

### Example 1: Construction of adenylyltransferase (GlnE) deficient strain

### <1> Preparation of plasmid for deletion of GlnE

The *glnE* sequence of the *Brevibacterium flavum* ATCC 14067 has' already been clarified (EP 1229121A2). Based on the reported nucleotide sequence, the primers shown in SEQ ID NOS: 5 and 6 were synthesized and used together with the chromosomal DNA of *Brevibacterium lactofermentum* ATCC 13869 strain as a template to amplify an internal sequence of *glnE* by the PCR method.

The chromosomal DNA of *Brevibacterium lactofermentum* ATCC 13869 strain was prepared by using Bacterial Genome DNA Purification Kit (Advanced Genetic Technologies Corp.). PCR was performed for 30 cycles each consisting of denaturation at 94°C for 30 seconds, annealing at 55°C for 15 seconds and extension at 72°C for 2 minutes using Pyrobest DNA Polymerase (Takara Shuzo).

The PCR product was purified in a conventional manner and blunt-ended by using Blunting Kit (Takara Shuzo). The blunt-ended PCR product was ligated to *HincII* site of pHSG299 using Ligation Kit (Takara Shuzo), and used to transform competent cells of *Escherichia coli* JM109 (Takara Shuzo). The cells were plated on L medium containing 10 µg/ml of IPTG, 40 µg/ml of X-Gal and 25 µg/ml of kanamycin and cultured overnight. Then, the emerged white colonies were picked up and separated into single colonies to obtain transformants.

Plasmids are prepared from the transformants by the alkali method, and structures of the plasmids were confirmed. A plasmid in which a partial fragment of *glnE* was inserted into the vector was designated as pΔATase-299.

The aforementioned pΔATase-299 does not include any sequence autonomously replicable in coryneform bacteria. Therefore, if a coryneform bacterium is transformed with this plasmid a strain in which this plasmid is incorporated into a chromosome by homologous recombination appears as a transformant, although it occurs at an extremely low frequency.

### <2> Introduction of pΔATase-299 into arginine repressor-deficient strain and evaluation of amino acid production

The arginine repressor-deficient strain obtained in Reference Example 1, 2256Δ*argR* strain, was transformed with the plasmid pΔATase-299 by the electric pulse method (see Japanese Patent Laid-open No. 2-207791), and transformants were obtained by using the kanamycin-resistance as a marker. A sequence around the *glnE* gene of each transformant was amplified by PCR, and disruption of the *glnE* gene was confirmed to obtain a strain in which *glnE* of 2256ΔargR was disrupted (2256ΔargRΔglnE). By using the obtained transformant 2256ΔargRΔglnE, culture for L-arginine and L-lysine production was performed as follows.

Cells of 2256ΔargRΔglnE obtained by culture on a CM2G plate medium containing 25 µg/ml of kanamycin were inoculated into a medium containing 40 g of glucose, 65 g of (NH₄)₂SO₄, 1 g of KH₂PO₄, 0.4 g of MgSO₄·7H₂O, 0.01 g of FeSO₄, 0.01 g of MnSO₄, 50 µg of VB₁-HCl, 50 µg of biotin, 45 mg (amount of N) of soybean hydrolysates and 50 g of CaCO₃ in 1 L of pure water (adjusted to pH 7.0 with KOH) and cultured at 31.5°C with shaking until the sugar in the medium was consumed.

After the completion of the culture, the accumulated L-arginine amount (Arg) in the culture broth was analyzed by liquid chromatography for appropriately diluted culture broth. The accumulated L-lysine amount (Lys) and accumulated L-glutamic acid amount (Glu) was analyzed for appropriately diluted culture broth by using Biotech Analyzer (Asahi Chemical Industry). The accumulated L-glutamine amount (Gln) and accumulated N-acetylglutamic acid amount was analyzed by liquid chromatography for appropriately diluted culture broth. The results are shown in Table 1.

The GS activity of each of the aforementioned strains was also measured. The GS activity was measured by adding a crude enzyme solution to a solution containing 100 mM imidazole-HCl (pH 7.0), 0.1 mM NH₄Cl, 1 mM MnCl₂, 1 mM phosphoenolpyruvic acid, 0.3 mM NADH, 10 U of lactate dehydrogenase, 25 U of pyruvate kinase, 1 mM ATP and 10 mM MSG and measuring variation of absorbance at 340 nm at 30°C referring to the method described in Journal of Fermentation and Bioengineering, Vol. 70, No. 3, 182-184, 1990. For the measurement of blank, the aforementioned reaction solution not containing MSG was used. The crude enzyme solution was prepared by separating cells from the aforementioned culture broth by centrifugation, washing the cells with 100 mM imidazole-HCl (pH 7.0), sonicating the cells and removing undisrupted cells by centrifugation. The protein concentration of the crude enzyme solution was quantified by using Protein Assay (Bio-Rad) with bovine serum albumin as a standard sample. The results are shown in Table 2.

**Table 1**

| Strain | Arg | Lys | Glu | Gln | N-Acetylglutamic acid |
|---|---|---|---|---|---|
| | (g/L) | (g/L) | (g/L) | (g/L) | (g/L) |
| 2256ΔargR | 2.28 | 0.81 | 0.32 | 0.19 | 0.0153 |
| 2256ΔargRΔglnE | 3.18 | 1.32 | 0.4 | 0.2 | 0.0381 |

**Table 2**

| Strain | GS activity (nmol/min/mg) |
|---|---|
| 2256ΔargR | 51.2 |
| 2256ΔargRΔglnE | 123.1 |

In the GlnE-deficient strain, improvement of L-arginine and L-lysine accumulations was observed compared with the parent strain, 2256ΔargR. The specific activity of GS was improved in the GlnE-deficient strain by about 2.4 times compared with the parent strain. Moreover, increase of N-acetylglutamic acid and glutamic acid, which are the precursors of L-arginine, was also observed.

The 2256ΔargRΔglnE strain was given with a private numver AJ110145, and it was deposited at the independent administrative agency, National Institute of Advanced Industrial Science and Technology, International Patent Organism Depository (postal code 305-5466, Tsukuba Central 6, 1-1 Higashi 1-Chome, Tsukuba-shi, Ibaraki-ken, Japan) on February 18, 2003 and given with an accession number of FERM P-19216. Then, it was converted to an international deposit based on the Budapest Treaty on February 19, 2004 and given an accession number of FERM BP-08630.

### Example 2: Evaluation of GS adenylylation site-modified strain

### <1> Construction of GlnA adenylylation site-modified plasmid

The adenylylation site of *glnA* gene product (GlnA) of coryneform bacteria had already been clarified (FEMS Microbiology Letters, 303-310, (173) 1999). Therefore, GlnA adenylylation site-modified strain was obtained by replacing the *glnA* gene on the chromosome with a *glnA* gene of which adenylylation site of GlnA was modified. Specific procedures will be described herein.

First, PCR was performed using chromosome DNA of *Brevibacterium* lactovermentum ATCC13869 strain as a template and the synthetic DNAs shown in SEQ ID NOS: 7 and 8 as primers to obtain an amplification product for the N-terminus side of the *glnA* gene. Separately, in order to obtain an amplification product for the C-termirius side of the *glnA* gene, PCR was performed using chromosome DNA of *Brevibacterium lactofermentum* ATCC13869 strain as a template and the synthetic DNAs shown in SEQ ID NOS: 9 and 10 as primers. Since mismatches were introduced into the sequences shown in SEQ ID NOS: 8 and 9, a mutation was introduced into the terminal portion of each of the amplification products. Specifically, the tyrosine residue at position 405 is replaced with an L-phenylalanine residue by this mutation.

Then, in order to obtain a *glnA* gene fragment introduced with a mutation, PCR was performed by using the aforementioned gene products for N- and C-terminus sides of *glnA* mixed in equimolar amounts as a template and the synthetic DNAs shown in SEQ ID NOS: 10 and 11 as primers to obtain a *glnA* gene amplification product introduced with a mutation at the adenylylation site. The PCR product was purified in a conventional manner, digested with *Hinc*II and inserted into the *Hinc*II site of pHSG299 (Takara Shuzo). This plasmid was designated as pGSA2.

Since the above pGSA2 does not contain a region that enables its autonomous replication in coryneform bacteria, when a coryneform bacterium is transformed with this plasmid, a strain in which the plasmid is incorporated into chromosome by homologous recombination is obtained as a transformant, although it occurs at an extremely low frequency.

### <2> Introduction of pGSA2 into arginine repressor-deficient strain and evaluation of amino acid production

The ArgR-deficient strain derived from *Brevibacterium lactofermentum* ATCC 13869 described in Reference Example 1, 2256ΔR, was transformed with the plasmid pGSA2 of a high concentration by the electric pulse method (see Japanese Patent Laid-open No. 2-207791), and transformants were obtained by using the kanamycin resistance as a marker. The *glnE* gene sequences of each transformant was determined, and a transformant in which the adenylylation site in the sequence was replaced with that region in *glnA* derived from pGSA2 was designated 2256ΔargRAde. By using the 2256ΔargR strain and 2256ΔargRAde strain, culture for L-arginine and L-lysine production and measurement of GS enzymatic activity were performed in the same manner as that described in Example 1, <2>. The results are shown in Tables 3 and 4.

**Table 3**

| Strain | Arg (g/L) | Lys (g/L) | Glu (g/L) | Gln (g/L) | N-Acetylglutamic acid (g/L) |
|---|---|---|---|---|---|
| 2256ΔargR | 2.28 | 0.81 | 0.32 | 0.19 | 0.0153 |
| 2256ΔargRAde | 2.88 | 1.48 | 0.49 | 0.31 | 0.314 |

**Table 4**

| Strain | GS activity (nmol/min/mg) |
|---|---|
| 2256ΔargR | 51.2 |
| 2256ΔargRAde | 135.1 |

In the 2256ΔargRAde strain, improvement of L-arginine and L-lysine accumulations was observed compared with 2256ΔargR. Moreover, increase of L-glutamic acid, L-glutamine and N-acetylglutamic acid, which are the precursors of L-arginine, was also observed. The specific activity of GS increased by about 2.6 times.

The 2256ΔargRAde strain was given with a private number of AJ110146, and it was deposited at the independent administrative agency, National Institute of Advanced Industrial Science and Technology, International Patent, Organism Depository (postal code 305-5466, Tsukuba Central 6, 1-1 Higashi 1-Chome, Tsukuba-shi, Ibaraki-ken, Japan) on February 18, 2003 and given with an accession number of FERM P-19217. Then, it was converted to an international deposit based on the Budapest Treaty on February 19, 2004, and given an accession number of FERM BP-08631.

### Example 3: Acquisition and evaluation of AmtR-deficient strain

### <1> Preparation of plasmid for deletion of AmtR

A plasmid for deleting the *amtR* gene product (AmtR) of coryneform bacteria was prepared as follows.

First, a chromosomal DNA of *Brevibacterium lactofermentum* ATCC 13869 strain was extracted and used as a template together with the synthetic DNAs shown in SEQ ID NOS: 13 and 14 as primers to perform PCR. The obtained DNA fragment was blunt-ended and inserted into pHSG299 (Takara Shuzo) at the *Hinc*II site. The obtained plasmid was designated pΔamtRT.

The aforementioned pΔamtR does not include any sequence enabling autonomous replication within a cell of coryneform bacteria. Therefore, if a coryneform bacterium is transformed with this plasmid, a strain in which this plasmid is incorporated into a chromosome by homologous recombination appears as a transformant, although it occurs at an extremely low frequency.

### <2> Introduction of pΔamtR into arginine repressor-deficient strain and evaluation of culture

The arginine repressor-deficient strain obtained in Reference Example 1, 2256ΔargR strain, was transformed with pΔamtR, and transformants were obtained by using the kanamycin-resistance as a marker.
A sequence around the *amtR* gene of each transformant was determined, and disruption of the *amtR* gene was confirmed to obtain an *amtR*-disrupted strain of 2256ΔargR (2256ΔargRΔamtR). By using the obtained transformant 2256ΔargRΔamtR, culture for L-arginine and L-lysine production was performed in the same manner as that described in Example 1, <2>. The results are shown in Tables 5 and 6.

**Table 5**

| Strain | Arg | Lys | Glu | Gln | N-Acetylglutamic acid |
|---|---|---|---|---|---|
| | (g/L) | (g/L) | (g/L) | (g/L) | (g/L) |
| 2256ΔargR | 2.28 | 0.81 | 0.32 | 0.19 | 0.0153 |
| 2256ΔargRΔamtR | 3.16 | 1.48 | 0.45 | 0.41 | 0.0296 |

**Table 6**

| Strain | GS activity (nmol/min/mg) |
|---|---|
| 2256ΔargR | 51.2 |
| 2256ΔargRΔamtR | 132.3 |

In the 2256ΔargRΔamtR strain, improvement of L-arginine and L-lysine accumulations was observed compared with 2256ΔargR. Moreover, accumulation of glutamic acid, N-acetylglutamic acid and glutamine, which are the precursors of L-arginine, was also observed. The specific activity of GS increased by about 2.6 times.

The 2256ΔargRΔamtR strain was given with a private number of AJ110144, and it was deposited at the independent administrative agency, National Institute of Advanced Industrial Science and Technology, International Patent Organism Depository (postal code 305-5466, Tsukuba Central 6, 1-1 Higashi 1-Chome, Tsukuba-shi, Ibaraki-ken, Japan) on February 18, 2003 and given with an accession number of FERM P-19215. Then, it was converted to an international deposit based on the Budapest Treaty on February 19, 2004, and given an accession number of FERM BP-08629.

### [Explanation of Sequence Listing]

SEQ ID NO: 1: Primer for *argR* gene amplification
SEQ ID NO: 2: Primer for *argR* gene amplification
SEQ ID NO: 3: Primer for amplification of a part of the plasmid containing the *argR* gene other than the *argR* gene ORF
SEQ ID NO: 4: Primer for amplification of a part of the plasmid containing the *argR* gene other than the *argR* gene ORF
SEQ ID NO: 5: Primer N for *glnE* disruption
SEQ ID NO: 6: Primer C for *glnE* disruption
SEQ ID NO: 7: GlnA adenylylation 1st PCR primer NN
SEQ ID NO 8: GlnA adenylylation 1st PCR primer NC
SEQ ID NO: 9: GlnA adenylylation 1st PCR primer CN
SEQ ID NO: 10: GlnA adenylylation 1st PCR primer CC
SEQ ID NO: 11: *glnA* 2nd PCR primer N
SEQ ID NO: 12: *glnA* 2nd PCR primer C
SEQ ID NO: 13: Primer N for *amtR* disruption
SEQ ID NO: 14: Primer C for *amtR* disruption
SEQ ID NO: 15: nucleotide sequence of *argR* gene
SEQ ID NO: 16: amino acid sequence encoded by the above DNA fragment
SEQ ID NO: 17: nucleotide sequence of *glnE* gene
SEQ ID NO: 18: amino acid sequence encoded by the above DNA fragment
SEQ ID NO: 19: nucleotide sequence of *glnA* gene
SEQ ID NO: 20: amino acid sequence encoded by the above DNA fragment
SEQ ID NO: 21: nucleotide sequence of *amtR* gene
SEQ ID NO: 22: aino acid sequence encoded by the above DNA fragment
SEQ ID NO: 23: ncleotide sequence of *glnB* gene
SEQ ID NO: 24: amino acid sequence encoded by the above DNA fragment

## Claims

1. A method for producing L-arginine or L-lysine, comprising the steps of
(a) culturing a coryneform bacterium in a medium to produce and
(b) accumulate L-arginine or L-lysine in the medium and
(c) collecting the L-arginine or L-lysine from the medium,
wherein said coryneform bacterium has an L-arginine- or L-lysine-producing ability and is modified so that glutamine synthetase activity is enhanced by
(i) modifying an adenylylation site of glutamine synthetase so that activity regulation of glutamine synthetase by adenylylation is reduced compared with that of a wild strain or unmodified strain of a coryneform bacterium, or eliminated,
(ii) disrupting a gene encoding the glutamine synthetase adenylyltransferase on a chromosome of said bacterium,
(iii) disrupting a gene encoding PII protein on a chromosome, wherein the gene encoding PII protein is selected from the group consisting of:
a) a DNA having the nucleotide sequence of SEQ ID NO: 23,
b) a DNA encoding a protein having PII activity and being hybridizable with the nucleotide sequence of SEQ ID NO:23 under stringent conditions comprising washing at 0.1 x SSC, 0.1 % SDS, at 60°C,
(iv) disrupting the *amtR* gene on a chromosome of said bacterium, wherein the amtR gene is selected from the group consisting of:
a) a DNA having the nucleotide sequence of SEQ ID NO: 21,
b) a DNA encoding a protein having AmtR activity and being hybridizable with the nucleotide sequence of SEQ ID NO:21 under stringent conditions comprising washing at 0.1 x SSC, 0.1 % SDS, at 60°C, or
(v) enhancing expression of a gene encoding glutamine synthetase by replacing an expression regulatory sequence with a stronger one,
and wherein said coryneform bacterium is modified so that the activity of the arginine repressor is reduced or eliminated as compared with a wild type strain or unmodified strain, wherein the arginine repressor is selected from the group consisting of:
a) a. protein encoded by a DNA having the nucleotide sequence of SEQ ID NO: 15,
b) a protein having arginine repressor activity and being encoded by a DNA that is hybridizable with the nucleotide sequence of SEQ ID NO:15 under stringent conditions comprising washing at 0.1 x SSC, 0.1 % SDS, at 60°C.

2. The method according to claim 1, wherein said gene encoding glutamine synthetase is selected from the group consisting of:
a) a DNA that encodes the amino acid sequence of SEQ ID NO: 20,
b) a DNA comprising the nucleotide sequence of SEQ ID NO: 19, and
c) a DNA that is hybridizable with the nucleotide sequence of SEQ ID NO:19 under stringent conditions comprising washing at 0.1 x SSC, 0.1 % SDS, at 60°C, and encodes a protein having glutamine synthetase activity.

## Patentansprüche

1. Verfahren zum Herstellen von L-Arginin oder L-Lysin, welches die Stufen umfasst, bei denen
(a) ein coryneformes Bakterium in einem Medium kultiviert wird, um L-Arginin oder L-Lysin zu produzieren, und
(b) L-Arginin oder L-Lysin in dem Medium angehäuft wird und
(c) das L-Arginin oder L-Lysin aus dem Medium gewonnen wird,
wobei das coryneforme Bakterium die Fähigkeit zur Produktion von L-Arginin oder L-Lysin aufweist und so modifiziert ist, dass die Glutaminssynthetaseaktivität erhöht ist, indem
(i) eine Adenylylieriungsstelle von Glutaminsynthetase modifiziert ist, so dass die Aktivitätsregulation von Glutaminsynthetase durch Adenylylierung im Vergleich zu derjenigen eines Wildtypstammes oder unmodifizierten Stammes eines coryneformen Bakteriums verringert oder ausgeschaltet ist,
(ii) ein für Glutaminsynthetase-Adenylyltransferase kodierendes Gen auf dem Chromosom des Bakteriums unterbrochen ist,
(iii) ein für das PII-Protein kodierende Gen auf einem Chromosom unterbrochen ist, wobei das für das PII-Protein kodierende Gen aus der Gruppe ausgewählt ist, bestehend aus:
a) einer DNA der Nukleotidsequenz der SEQ ID NO:23,
b) einer für ein Protein mit PII-Aktivität kodierendes Gen, das mit der Nukleotidsequenz der SEQ ID NO:23 unter stringenten Bedingungen, die das Waschen bei 0,1 x SSC, 0,1 % SDS bei 60°C umfassen, hybridisierbar ist,
(iv) das amtR-Gen auf einem Chromosom des Bakteriums unterbrochen ist, wobei das amtR-Gen aus der Gruppe ausgewählt ist, bestehend aus:
a) einer DNA der Nukleotidsequenz der SEQ ID NO:21,
b) einer für ein Protein mit AmtR-Aktivität kodierenden DNA, die mit der Nukleotidsequenz der SEQ ID NO:21 unter stringenten Bedingungen, die das Waschen bei 0,1 x SSC, 0,1 % SDS bei 60°C umfassen, hybridisierbar ist, oder
(v) die Expression eines für Glutaminsynthetase kodierenden Gens erhöht ist, indem eine Expressionsregulationssequenz durch eine stärkere ersetzt ist,
und wobei das coryneforme Bakterium so modifiziert ist, dass die Aktivität des Argininrepressors im Vergleich zu einem Wildtypstamm oder unmodifizierten Stamm verringert oder ausgeschaltet ist, wobei der Argininrepressor aus der Gruppe ausgewählt ist, bestehend aus:
a) einem durch eine DNA der Nukleotidsequenz der SEQ ID NO:15 kodierten Protein,
b) einem Protein mit Argninrepressoraktivität, das durch eine DNA kodiert wird, die mit der Nukleotidsequenz der SEQ ID NO:15 unter stringenten Bedingungen, die das Waschen bei 0,1 x SSC, 0,1 % SDS bei 60°C umfassen, hybridisierbar ist.

2. Verfahren nach Anspruch 1, wobei das für Glutaminsynthetase kodierende Gen aus der Gruppe ausgewählt ist, bestehend aus:
a) einer die Aminosäuresequenz der SEQ ID NO:20 kodierenden DNA,
b) einer die Nukleotidsequenz der SEQ ID NO:19 umfassenden DNA und
c) einer DNA, die mit der Nukleotidsequenz der SEQ ID NO:19 unter stringenten Bedingungen, die das Waschen bei 0,1 x SSC, 0,1 % SDS bei 60°C umfassen, hybridisierbar ist und für ein Protein mit Glutaminsynthetaseaktivität kodiert.

## Revendications

1. Procédé de production de L-arginine ou de L-lysine, comprenant les étapes consistant à
(a) cultiver une bactérie corynéforme dans un milieu pour produire et
(b) accumuler une L-arginine ou une L-lysine dans le milieu et
(c) collecter la L-arginine ou la L-lysine à partir du milieu,
dans lequel ladite bactérie corynéforme a une capacité de production de L-arginine ou de L-lysine et est modifiée de sorte que l'activité glutamine synthétase soit renforcée en
(i) modifiant un site d'adénylylation de la glutamine synthétase de sorte que la régulation de l'activité de la glutamine synthétase par adénylylation soit réduite en comparaison avec celle d'une souche sauvage ou d'une souche non modifiée d'une bactérie corynéforme, ou éliminée,
(ii) dissociant un gène codant pour la glutamine synthétase adénylyltransférase sur un chromosome de ladite bactérie,
(iii) dissociant un gène codant pour la protéine PII sur un chromosome, le gène codant pour la protéine PII étant choisi dans le groupe constitué de :
a) un ADN ayant la séquence nucléotidique de SEQ ID N° : 23,
b) un ADN codant pour une protéine ayant une activité PII et pouvant être hybridé avec la séquence nucléotidique de SEQ ID N° : 23 dans des conditions stringentes comprenant un lavage à 0,1 x SSC, SDS à 0,1 %, à 60 °C,
(iv) dissociant le gène *amtR* sur un chromosome de ladite bactérie, le gène amtR étant choisi dans le groupe constitué de :
a) un ADN ayant la séquence nucléotidique de SEQ ID N° : 21,
b) un ADN codant pour une protéine ayant une activité AmtR et pouvant être hybridé avec la séquence nucléotidique de SEQ ID N° : 21 dans des conditions stringentes comprenant un lavage à 0,1 x SSC, SDS à 0,1 %, à 60 °C, ou
(v) renforçant l'expression d'un gène codant pour la glutamine synthétase en remplaçant une séquence de régulation de l'expression par une séquence plus forte,
et dans lequel ladite bactérie corynéforme est modifiée de sorte que l'activité du répresseur d'arginine soit réduite ou éliminée en comparaison avec une souche de type sauvage ou une souche non modifiée, le répresseur d'arginine étant choisi dans le groupe constitué de :
a) une protéine codée par un ADN ayant la séquence nucléotidique de SEQ ID N° : 15,
b) une protéine ayant une activité répresseur d'arginine et étant codée par un ADN qui peut être hybridé avec la séquence nucléotidique de SEQ ID N° :15 dans des conditions stringentes comprenant un lavage à 0,1 x SSC, SDS à 0,1 %, à 60 °C.

2. Procédé selon la revendication 1, dans lequel ledit gène codant pour la glutamine synthétase est choisi dans le groupe constitué de :
a) un ADN qui code pour la séquence d'acides aminés de SEQ ID N° : 20,
b) un ADN comprenant la séquence nucléotidique de SEQ ID N° : 19, et
c) un ADN qui peut être hybridé avec la séquence nucléotidique de SEQ ID N° : 19 dans des conditions stringentes comprenant un lavage à 0,1 x SSC, SDS à 0,1 %, à 60 °C, et qui code pour une protéine ayant une activité glutamine synthétase.
